# EUROPEAN PATENT SPECIFICATION

(11) **EP 0 801 642 B1**
(45) Date of publication and mention of the grant of the patent: **20.12.2000**
(21) Application number: 96935567.6
(22) Date of filing: 01.11.1996
(51) Int. Cl.: C07C 307/02, A61K 31/27

(54) **NOVEL SULFAMATE COMPOUND CONTAINING N-SUBSTITUTED CARBAMOYL GROUP AND METHOD FOR PREPARING THE SAME**
NEUE SULFAMATE MIT N-SUBSTITUIERTEN CARBAMOYL-GRUPPEN UND VERFAHREN ZU DEREN HERSTELLUNG
NOUVEAU COMPOSE DE SULFAMATE CONTENANT UN GROUPE CARBAMYLE N-SUBSTITUE ET PROCEDE DE PREPARATION D'UN TEL COMPOSE

(30) Priority: 02.11.1995 KR 1995394; 28.10.1996 KR 1996490
(43) Date of publication of application: 22.10.1997
(73) Proprietor: YUKONG LIMITED, Yongdungpo-ku, Seoul 150-010 (KR)
(72) Inventor: CHOI, Yong, Moon, Towaco, NJ 07082 (US); HAN, Dong, Il, Taejon 305-390 (KR); KIM, Hyung, Cheol, Taejon 305-390 (KR)
(74) Representative: Koch, Gustave
(86) International application number: KR9600190
(87) International publication number: WO9716418

(56) References cited:
- GB-A- 1 524 727
- US-A- 4 792 569
- US-A- 5 194 446
- CHEMICAL ABSTRACTS, Vol. 71, No. 3, 21 July 1969, (Columbus, Ohio, USA), pages 267-268, Abstract No. 12494t, LUDWIG B.J. et al., "Carbamate Derivatives Related to Meprobamate"; & MED. CHEM. 1969, 12(3), 462-72.

## Description

The present invention relates to pharmaceutically useful sulfamate compounds containing N-substituted carbamoyl group and, more particularly, to N,N'-substituted carbamoyl-2-aryl propanol sulfamates including their racemates and (R)- and (S)-optical isomers, useful for the prophylaxis and treatment of the disorders of the central nervous system. Also, the present invention is concerned with methods for preparing the same.

### Description of the Prior Art

Sulfamate compounds are well known to be useful as medicines for controlling various central nervous system (CNS) disorders, especially as antiepileptic.

As a prior art relating to these compounds, fructopyranose sulfamate compounds are reported in J. Med. Chem. 30, 880- 887(1987), together with their pharmaceutical effects. Other pharmaceutically useful sulfamates are also known. For example, sorbopyranose sulfamates and penethylsulfamates are disclosed in PCT WO 14827 and U. S. Pat. No. 4,792,569, respectively.

These compounds have effectively been used as therapeutical medicines for managing CNS diseases, such as an antiepileptic. Active research and development efforts have been and continue to be directed to the application of sulfamate compounds for CNS disorders.

### SUMMARY OF THE INVENTION

As a result of intensive and thorough research for the sulfamate derivatives of 2-aryl-1,3-propanediol, the present inventors found that the compounds introduced with N-substituted carbamoyl group are pharmaceutically useful in prophylaxis and treatment of CNS disorders.

Accordingly, it is an objective of the present invention to provide novel N-substituted carbamoyl -containing sulfamale compounds effective for the prophylaxis and treatment of CNS disorders. These sulfamate compounds comprise racemates represented by the following Formula I and their (R)- and (S)-optical isomers represented by the following Formulas II and III, respectively: 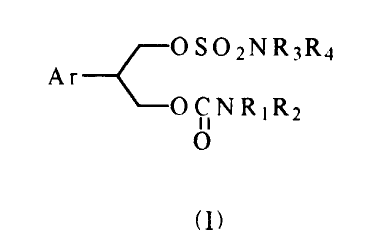 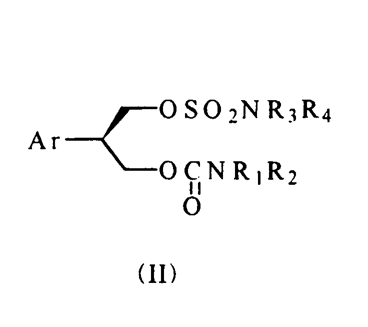 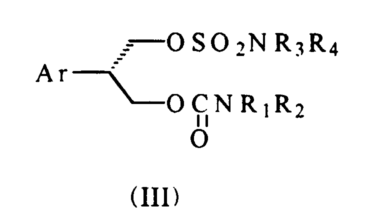 wherein,
Ar is represented by the following formulas; 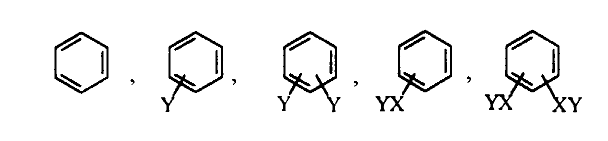
Y is selected from the group consisting of halogens such as F, Cl, Br and I, trifluoromethyl and alkyl groups containing 1 to 3 carbon atoms when Y alone binds to benzene ring and from the group consisting of trifluoromethyl and alkyl groups containing 1 to 3 carbon atoms when Y binds to X which is O or S; and
R₁, R₂, R₃ and R₄, identical or different, each are selected from the group consisting of hydrogen, linear or branched alkyl groups containing 1 to 16 carbon atoms, cyclic alkyl groups containing 3 to 16 carbon atoms and aryl groups containing 6 to 8 carbon atoms, and NR₁R₂ and NR₃R₄, identical or different, each may form a 3 to 7-membered aliphatic cyclic compound together with another nitrogen atom or oxygen atom.

Because the N,N'-substituted carbamoyl 2-aryl propanol sulfamate represented by Formula I has a chiral center at the benzyl position, they may be in either (R)- or (S)-optical isomer. In vivo, an optical isomer of one compound may exhibit even better pharmaceutical effect than other optical isomers, and many examples of the optical effect have been reported. Recent trend is to use optical isomers to develop new medicines. Thus, it is very important to separate the racemic mixture of one compound into respective optical isomers and apply them for pharmacology. Based upon this fact, the present inventors found that their (R)- and (S)-optical isomers are very effective for the prophylaxis and treatment of the disorders of the central nervous system, especially as antiepileptic, acute ischemic stroke and neuroprotective.

It is another objective of the present invention to provide methods for preparing the N,N'-substituted carbamoyl-2-aryl propanol sulfamate racemates and their pure (R)- and (S)-optical isomers.

It is a further objective of the present invention to provide the intermediates useful for producing the N,N-substituted carbamoyl-2-aryl propanol sulfamate racemates represented by Formula I, including 3-N-substituted carbamoyl-2-aryl propanol acetate represented by the following formula IV: 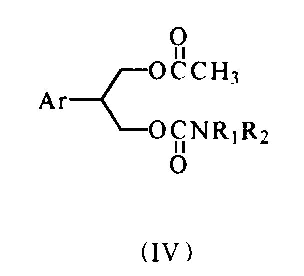 wherein Ar, R₁ and R₂ are as defined above, 3-N-substituted carbamoyl-2-aryl propanol represented by the following formula V: 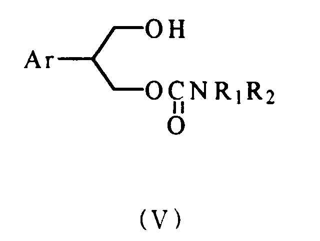 wherein Ar, R₁ and R₂ are as defined above, 3-acetoxy-2- aryl propanol sulfamate represented by the following formula VI: 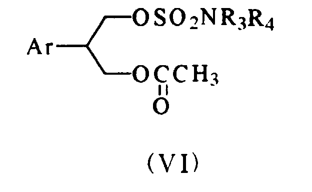 wherein Ar, R₃ and R₄ are as defined above, 2-aryl-1,3-propandiol monosulfamate represented by the following formula VII: 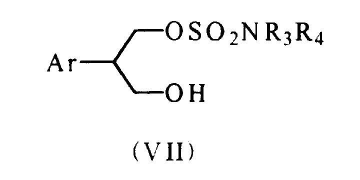 wherein Ar, R₃ and R₄ are as defined above; the intermediates useful for producing the (R)-N,N'-substituted carbamoyl-2-aryl propanol sulfamate represented by Formula II including (S)-3-N-substituted carbamoyl-2-aryl propanol acetate represented the following formula VIII: 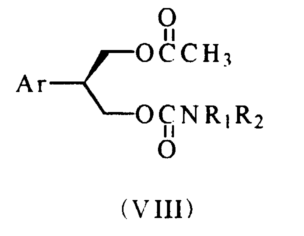 wherein Ar, R₁ and R₂ are as defined above with the proviso that R₁ and R₂ are not hydrogen atoms simultaneously, and (S)-3-N-substituted carbamoyl-2-aryl propanol represented by the following formula IX: 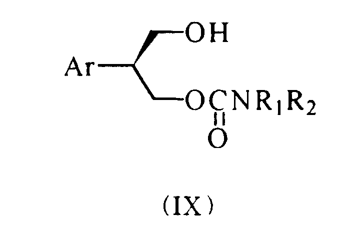 wherein Ar, R₁ and R₂ are as defined above with the proviso that R₁ and R₂ are not hydrogen atoms simultaneously; and the intermediates useful for producing the (S)-N,N'-substituted carbamoyl-2-aryl-propanol sulfamate represented by Formula III including the (S)-3-acetoxy-2-aryl-1,3-propandiol sulfamate represented by the following formula X: 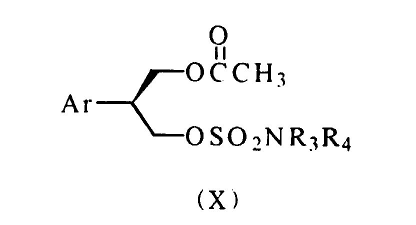 wherein Ar, R₃ and R₄ are as defined above, the (S)-2-aryl-1,3-propandiol monosulfamate represented by the following formula XI: 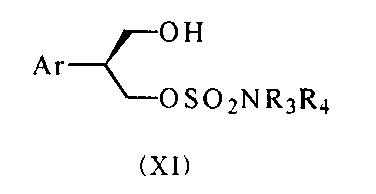 wherein Ar, R₃, and R₄ are as defined above, (R)-3-N-substituted carbamoyl-2-aryl propanol acetate represented by the following formula XII: 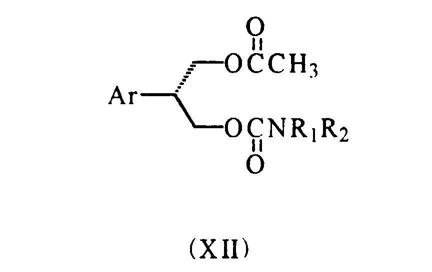 wherein Ar, R₁ and R₂ are as defined above with the proviso that R₁ and R₂ are not hydrogen atoms simultaneously, and (R)-3-N-substituted carbamoyl-2-aryl propanol represented by the following structural formula XIII: 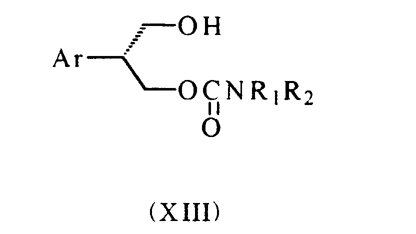 wherein Ar, R₁ and R₂ are as defined above with the proviso that R₁ and R₂ are not hydrogen atoms simultaneously.

For accomplishing the above objectives, there are provided the N,N'-substituted carbamoyl-2-aryl propanol sulfamate racemates represented by the following formula I: 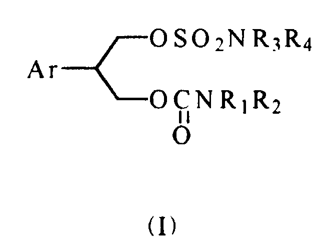 wherein
Ar is represented by the following formulas; 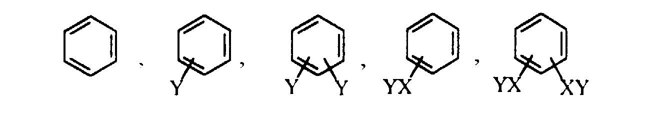
Y is selected from the group consisting of halogens such as F, Cl, Br and I, trifluoromethyl and alkyl groups containing 1 to 3 carbon atoms when Y alone binds to benzene ring and from the group consisting of trifluoromethyl and alkyl groups containing 1 to 3 carbon atoms when Y binds to X which is O or S; and
R₁, R₂, R₃ and R₄, identical or different, each are selected from the group consisting of hydrogen, linear or branched alkyl groups containing 1 to 16 carbon atoms, cyclic alkyl groups containing 3 to 16 carbon atoms and aryl groups containing 6 to 8 carbon atoms, and NR₁R₂ and NR₃R₄, identical or different, each may form a 3 to 7-membered aliphatic cyclic compound together with another nitrogen atom or oxygen atom.

The N,N-substituted carbamoyl-2-aryl propanol sulfamate compounds represented by Formula I can be prepared by the following three methods, in accordance with the present invention.

A detailed description will be given of a first method below, in conjunction with reaction schemes.

First, the 3-acetoxy-2-aryl propanol of Formula XIV is reacted with carbonyl diimidazole in dichloromethane solution to give the 3-imidazolyl carbonyloxy-2-aryl propanol acetate of Formula XV, which is, without purification, reacted with the substituted amine of Formula XVI to produce the 3-N-substituted carbamoyl-2-aryl propanol acetate of Formula IV, as shown in Reaction Scheme I: 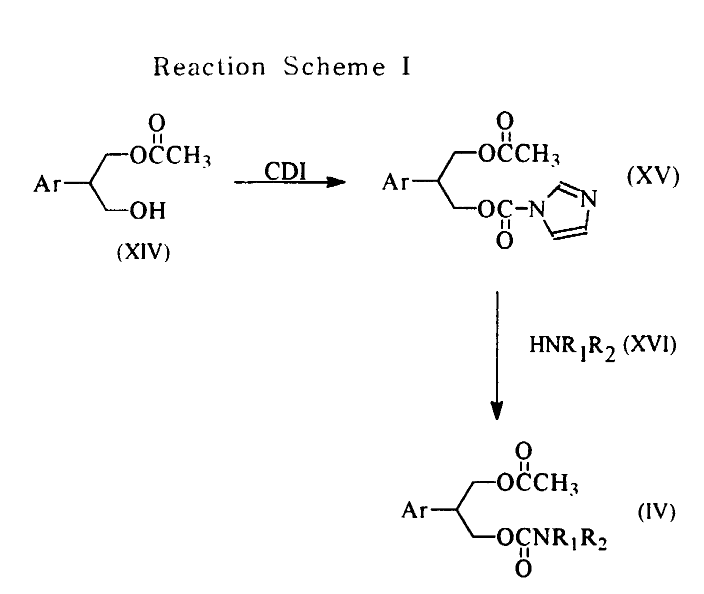 wherein Ar, R₁ and R₂ each are as defined above.

Detailed conditions for the above reactions are as follows; The compound of Formula XIV, the starting material, is reacted preferably at an amount of about 0.1 to 2.0 moles with 1.1 to 2.5 equivalents of carbonyl diimidazole when considering yield and economical aspects. It is preferable that the former reaction of Reaction Scheme 1 is carried out at a temperature of -5 to 40°C. For example, if the reaction is performed at a temperature less than -5°C, the progress of the reaction is very slow. On the other hand, if it is carried out at a temperature higher than 40°C, the yield decreases due to side reactions. Useful solvents in this reaction include low hydrocarbon halide solvents, such as methylene chloride and chloroform, ethereal solvents, such as ethyl ether and tetrahydrofuran, and acetonitrile with preference to methylene chloride, chloroform and tetrahydrofuran.

In the preparation reaction of the compound of Formula IV from the compound of Formula XV, the substituted amine is used at an amount of 1.0 to 5.0 equivalents, in consideration of rapid reaction and after-treatment. This reaction is executed at a temperature ranging from 0 to 30°C in a solvent selected from tetrahydrofuran and methylene chloride.

Next, the 3-N-substituted carbamoyl-2-aryl propanol acetate of Formula IV is subjected to transesterification in an alcohol solvent in the presence of a base catalyst, to give the 3-N-substituted carbamoyl-2-aryl propanol of the following formula V, as shown in Reaction Scheme II: 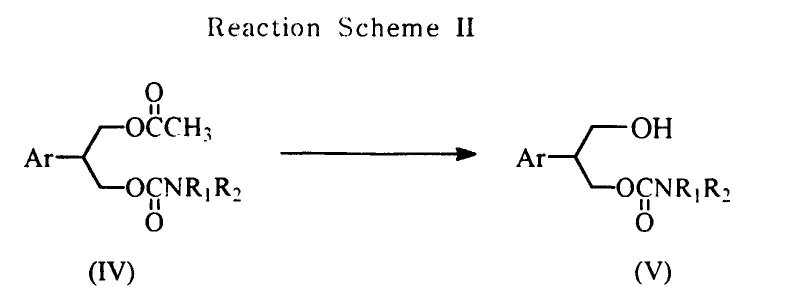 wherein Ar, R₁ and R₂ each are as defined above.

In the transesterification, the 3-N-substituted carbamoyl -2-aryl propanol acetate of Formula IV is used at an amount of 0.1 to 2.0 moles. As the base catalyst, sodium hydroxide, potassium hydroxide, sodium carbonate, lithium hydroxide, potassium carbonate, sodium bicarbonate or potassium cyanide is used at an amount of 0.1 to 1.0 equivalent at a temperature of 0 to 30°C. Useful solvents include methyhalcohol, ethylalcohol and propylalcohol. Preferred is methylalcohol when considering the reaction yield and the removal convenience after completion of the reaction. Attention must be paid to the point that the base catalyst remained in the reaction mixture is required to be inactivated after the reaction. If the active base catalyst is remained, a reverse reaction occurs during after-treatment, returning the product into the starting material. For the inactivation of the base catalyst, a proper acidic material is added to the reaction mixture after the reaction has been completed. Preferred is 1N hydrochloric acid solution or saturated ammonium chloride solution.

In subsequence, the compound of Formula V is reacted with sulfamoyl chloride in the presence of a base catalyst, to prepare the N,N'-substituted carbamoyl-2-aryl propanol sulfamate. This reaction is depicted in Reaction Scheme III: 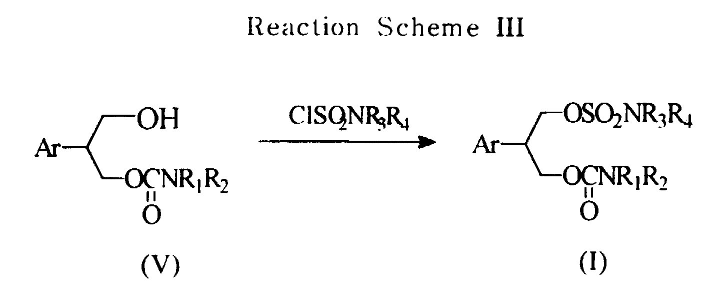 wherein Ar, R₁, R₂, R₃ and R₄ are as defined above.

The 3-N-substituted carbamoyl-2-aryl propanol represented by Formula V is used at an amount of 0.1 to 2.0 moles. As the base catalyst, triethylamine, pyridine, antipyrin, or diisopropylethylamine is employed. Available reaction solvents include amide solvents, such as dimethylformamide, ethereal solvents, such as ethyl ether and tetrahydrofuran, and acetonitrile with preference to tetrahydrofuran and acetonitrile. It is preferable that this reaction is carried out at a temperature of -10 to 40°C. For example, if the reaction temperature is maintained below -10 °C, the progress of the reaction is very slow. On the other hand, if the reaction is carried out at a temperature higher than 40°C, the yield lowers by the production of unknown by-products. When considering reaction progress, convenience in after-treatment and economical aspects, it is preferable that the base catalyst and the sulfamoyl chloride are used at an amount ranging from 2.0 to 4.0 equivalents and from 1.5 to 3.0 equivalents, respectively.

Following is a second method for preparing the compound of Formula I.

Initially, the 3-acetoxy-2-aryl propanol represented by Formula XIV is reacted with sulfamoyl chloride in an acetonitrile solvent in the presence of a base catalyst, to give the 3-acetoxy-2-aryl propanol sulfamate of Formula VI, as shown in Reaction Scheme IV: 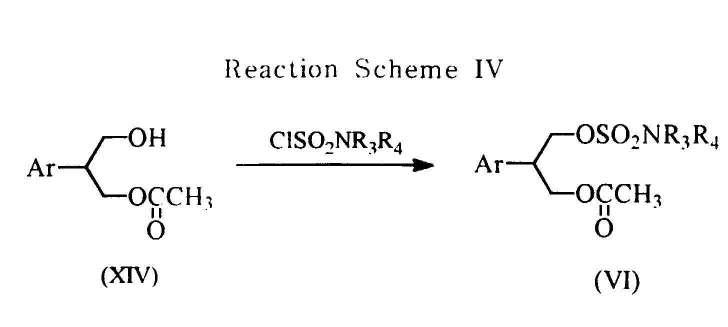 wherein Ar, R₃ and R₄ each are as defined above.

The 3-acetoxy-2-aryl propanol of Formula XIV is used at an amount of 0.1 to 2.0 moles. As the base catalyst, triethylamine, pyridine, antipyrin, or diisopropylethylamine is employed. Available reaction solvents include amide solvents, such as dimethylformamide, ethereal solvents, such as ethyl ether and tetrahydrofuran, and acetonitrile. Of them tetrahydrofuran and acetonitrile are preferred by virtue of their being easily removed after the reaction. It is preferable that this reaction is carried out at a temperature of -10 to 40°C. For example, if the reaction is executed at too low temperature, the reaction is very slow. On the other hand, if the reaction is carried out at a temperature higher than 40°C, the yield lowers by the production of unknown by-products. When considering reaction progress, convenience in after-treatment and economical aspects, it is preferable that the base catalyst and the sulfamoyl chloride are used at an amount ranging from 2.0 to 4.0 equivalents and from 1.5 to 3.0 equivalents, respectively.

Thereafter, using a base catalyst, the 3-acetoxy-2-aryl propanol sulfamate represented by Formula VI is subjected to transesterification in an alcohol solvent, to give the 2-aryl-1,3-propandiol monosulfamate of Formula VII, as depicted in Reaction Scheme V: 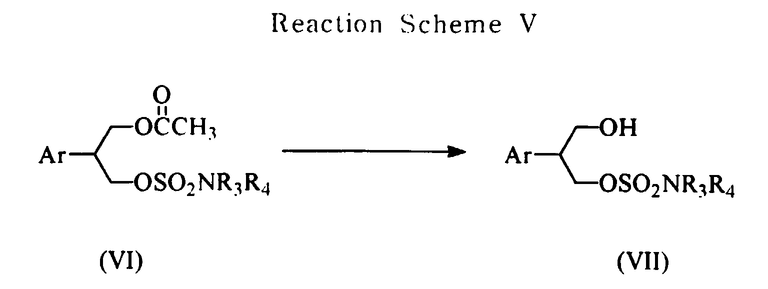 wherein Ar, R₃ and R₄ each are as defined above.

In such transesterification, the 3-acetoxy-2-aryl propanol sulfamate of Formula VI is used at an amount of 0.1 to 2.0 moles. As the base catalyst, sodium hydroxide, potassium hydroxide, lithium hydroxide, sodium carbonate, sodium bicabonate, potassium carbonate, or potassium cyanide is used at an amount of 0.1 to 1.0 equivalent at a temperature of 0 to 30°C. Useful solvents include methylalcohol, ethylalcohol and propylalcohol. Preferred is methylalcohol when considering the reaction yield and the removal convenience after completion of the reaction. As the first method, the base catalyst remained in the reaction mixture is required to be inactivated after completion of the transesterification. For this, 1N hydrochloric acid solution or saturated ammonium chloride solution is preferably used.

In a subsequent procedure, the 2-aryl-1,3-propandiol monosulfamate of Formula VII obtained is reacted with carbonyldiimidazole in dichloromethane solvent to give the 3-imidazolyl carbonyloxy-2-arylpropanol sulfamate represented by formula XVII, which is, without purification, reacted with the substituted amine of Formula XVI to prepare the N,N'-substituted carbamoyl-2-aryl propanol sulfamate compounds of Formula I, the objective compound, as depicted in Reaction Scheme VI:. 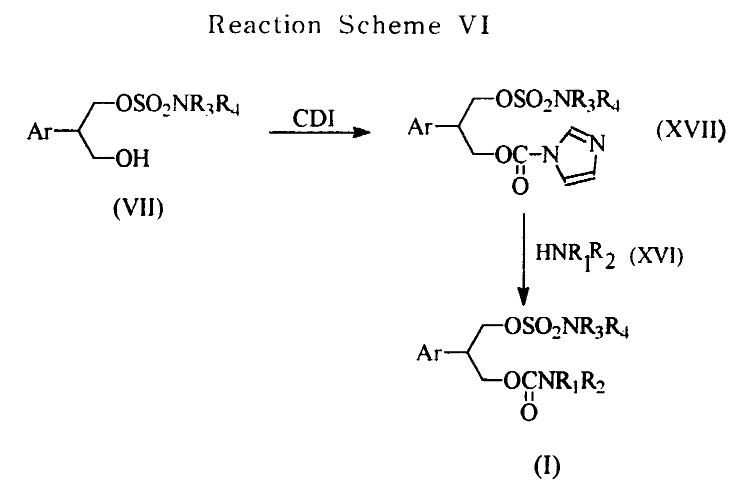 wherein Ar, R₁, R₂, R₃ and R₄ each are as defined above.

About 0.1 to 2.0 moles of the compound of Formula VII is preferably reacted with 1.1 to 2.5 equivalents of carbonyl diimidazole when considering yield and economical aspects. It is preferable that the former reaction of Reaction Scheme VI is carried out at a temperature of -5 to 40°C. For example, if the reaction is performed at a temperature less than -5°C, the reaction is very slow. On the other hand, if it is carried out at a temperature higher than 40°C, the yield decreases due to side reactions. Useful solvents for this reaction include low hydrocarbon halide solvents, such as methylene chloride and chloroform, ethereal solvents, such as ethyl ether and tetrahydrofuran, and acetonitrile with preference to methylene chloride, chloroform and tetrahydrofuran.

In the reaction of preparing the compound of Formula I from the compound of Formula XVII, the substituted amine is used at an amount of 1.0 to 5.0 equivalents at a temperature ranging from 0 to 30°C in a solvent selected from tetrahydrofuran and methylene chloride.

In a third method for preparing the compound of Formula I, the 2-aryl-1,3-propandiol monosulfamate of Formula VII is reacted with isocyanate represented by Formula XVIII in a hydrocarbon halide or ethereal hydrocarbon solvent, to prepare the N,N'-substituted carbamoyl-2-aryl-1,3-propandiol sulfamate compound of Formula I, as shown in Reaction Scheme VII: 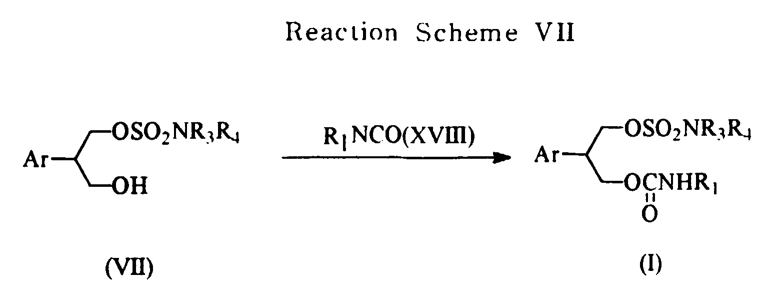 wherein Ar, R₁, R₃ and R₄ each are as defined above.

As shown in Reaction Scheme VII, the 2-aryl-1,3 -propandiol monosulfamate of Formula VII, the starting material, is reacted preferably at an amount of about 0.1 to 2.0 moles with 1.0 to 2.0 equivalents of isocyanate when considering reaction progress and economical aspects. It is preferable that this reaction is carried out at a temperature of 0 to 80°C. For example, if the reaction is performed at a temperature less than 0°C, the reaction is not accomplished. On the other hand, if it is carried out at a temperature higher than 80°C, too much by-products are produced by the reaction of isocyanate itself. Useful solvents for this reaction include low hydrocarbon halide solvents, such as methylene chloride and chloroform, ethereal solvents, such as ethyl ether and tetrahydrofuran, and aromatic hydrocarbon solvents, such as benzene and toluene with preference to methylene chloride, chloroform, benzene and toluene.

In accordance with the present invention, the compound of Formula II is prepared by following pathway described below.

This preparation starts with (R)-3-acetoxy-2-aryl propanol as shown in Reaction Scheme VIII: 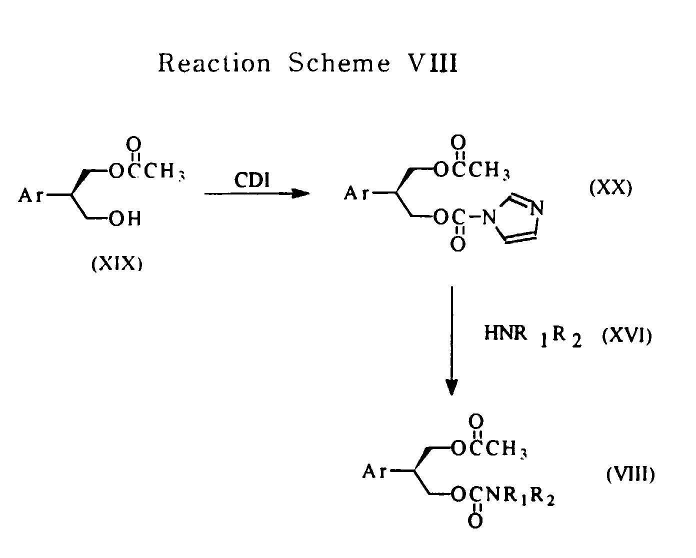 wherein Ar, R₁ and R₂ each are as defined above.

(R)-3-acetoxy-2-aryl propanol of Formula XIX is reacted with carbonyl diimidazole in dichloromethane solvent, to give (S)-3-imidazolyl carbonyloxy-2-aryl propanol acetate which is, without purification, reacted with the substituted amine of Formula XVI, to produce the (S)-3-N-substituted carbamoyl-2-aryl propanol acetate of Formula VIII.

The compound of Formula XIX, the starting material, is reacted preferably at an amount of about 0.1 to 2.0 moles with 1.1 to 2.5 equivalents of carbonyl diimidazole when considering yield and economical aspects. It is preferable that the former reaction of Reaction Scheme VIII is carried out at a temperature of -5 to 40°C. For example, if the reaction temperature is below -5°C, the reaction is very slow. On the other hand, if the former reaction is carried out at a temperature higher than 40°C, the yield decreases by side reactions. Useful solvents for this reaction include low hydrocarbon halide solvents, such as methylene chloride and chloroform, ethereal solvents, such as ethyl ether and tetrahydrofuran, and acetonitrile with preference to methylene chloride, chloroform and tetrahydrofuran.

For the preparation reaction of the compound of Formula VIII from the compound of Formula XX, the substituted amine is used at an amount of 1.0 to 5.0 equivalents. This reaction is executed at a temperature ranging from 0 to 30°C in a solvent such as tetrahydrofuran and methylene chloride.

Then, the (S)-3-N-substituted carbamoyl-2-aryl propanol acetate of Formula VIII is subjected to transesterification to prepare (S)-3-N-substituted carbamoyl-2-aryl propanol of Formula IX in an alcohol solvent in the presence of a base catalyst, as shown in Reaction Scheme IX: 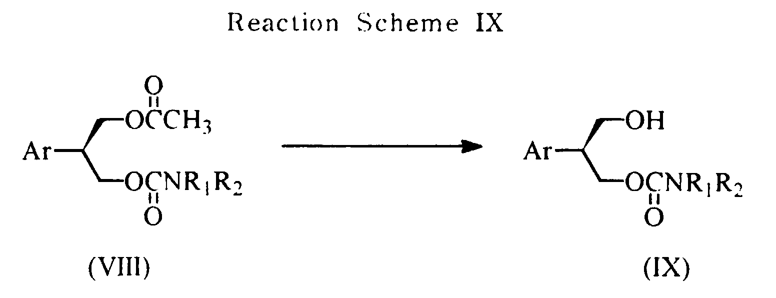 wherein Ar, R₁ and R₂ each are as defined above.

In the transesterification, the (S)-3-N-substituted carbamoyl-2-aryl propanol acetate of Formula VIII is used at an amount of 0.1 to 2.0 moles. As the base catalyst, sodium hydroxide, potassium hydroxide, lithium hydroxide, potassium carbonate, or potassium cyanide is used at an amount of 0.1 to 1.0 equivalent at a temperature of 0 to 30 °C. Useful solvents include methylalcohol, ethylalcohol and propylalcohol. Preferred is methylalcohol when considering the reaction yield and the removal convenience after completion of the reaction.

Also, as mentioned in the first and the second methods for the preparation of the compounds of Formula I, it is needed that the base catalyst remained in the reaction mixture should be inactivated after the reaction. For this, IN hydrochloric acid solution or saturated ammonium chloride solution is preferred.

In subsequence, the (S)-3-N-substituted carbamoyl-2-aryl propanol of Formula IX is reacted with sulfamoyl chloride in the presence of a base catalyst, to prepare the N,N'-substituted carbamoyl-2-aryl propanol sulfamate of Formula II. This reaction is depicted in Reaction Scheme X: 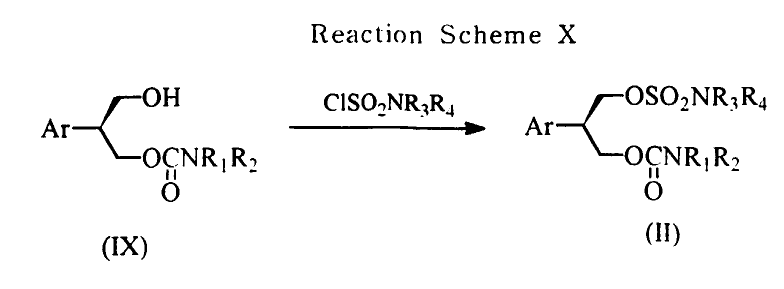 wherein Ar, R₁, R₂, R₃, and R₄ are as defined above.

The (S)-3-N-substituted carbamoyl-2-aryl propanol represented by Formula IX is used at an amount of 0.1 to 2.0 moles while the sulfamoyl chloride at an amount of about 1.0 to 3.0 equivalents. As the base catalyst, triethylamine, pyridine, antipyrin, or diisopropylethylamine is employed. When considering reaction progress, convenience in after-treatment and economical aspects, it is preferable that the base catalyst is used at an amount ranging from 2.0 to 4.0 equivalents. Available reaction solvents include amide solvents, such as dimethylformamide, ethereal solvents, such as ethyl ether and tetrahydrofuran, and acetonitrile with preference to tetrahydrofuran and acetonitrile. It is preferable that this reaction is carried out at a temperature of -10 to 40°C. For example, if the reaction temperature is maintained below -10°C, the reaction is significantly slow. On the other hand, if the reaction is carried out at a temperature higher than 40°C, the yield decreases by the production of unknown by-products.

There are two pathways for preparing the compound of Formula III, in accordance with the present invention.

In a first pathway, an (R)-3-acetoxy-2-aryl propanol of Formula (XIX) is used as the starting material, as shown in Reaction Scheme XI: 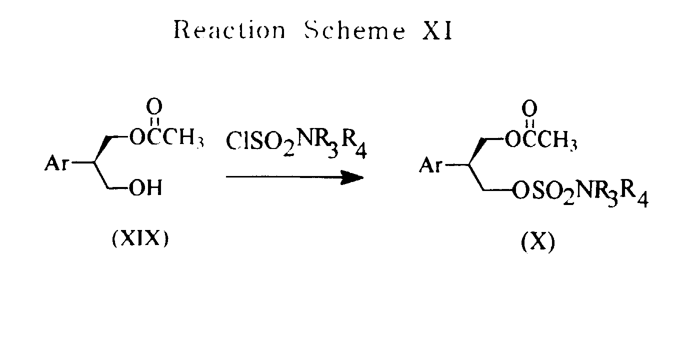 wherein Ar, R₃ and R₄ each are as defined above.

The (R)-3-acetoxy-2-aryl propanol of Formula XIX is reacted with sulfamoyl chloride in an acetonitrile solvent in the presence of a base catalyst, to give an (S)-3-acetoxy-2-aryl-1,3-propandiol sulfamate of Formula X.

The (R)-3-acetoxy-2-aryl propanol of Formula XIX is used at an amount of 0.1 to 2.0 moles. As the base catalyst, triethylamine, pyridine, antipyrin, or diisopropylethyl amine is employed. When considering reaction progress, convenience in after-treatment and economical aspects, it is preferable that the base catalyst is used at an amount ranging from 2.0 to 4.0 equivalents. Available reaction solvents include amide solvents, such as dimethylformamide, ethereal solvents, such as ethyl ether and tetrahydrofuran, and acetonitrile. Of them tetrahydrofuran and acetonitrile are preferred.

It is preferable that this reaction is carried out at a temperature of -10 to 40°C. For example, if the reaction is executed at too low temperature, the reaction is very slow. On the other hand, if the reaction is carried out at a temperature higher than 40°C, the yield decreases by the production of unknown by-products.

Thereafter, using a base catalyst, the (S)-3-acetoxy-2-aryl-1,3-propandiol sulfamate of Formula X is subjected to transesterification in an alcohol solvent, to give the (S)-2-aryl-1,3-propandiol monosulfamate of Formula XI, as depicted in Reaction Scheme XVI: 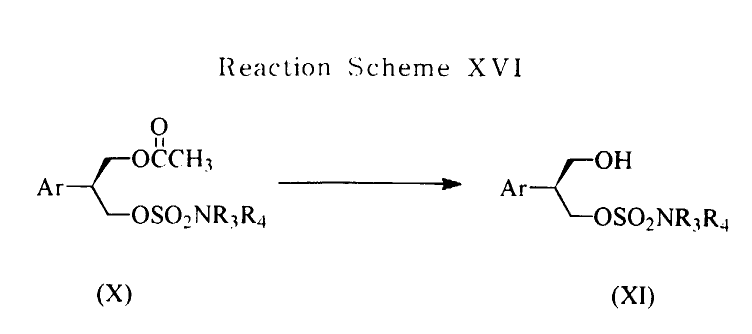 wherein Ar, R₃ and R₄ each are as defined above.

In this transesterification, the (S)-3-acetoxy-2-aryl-1,3-propandiol sulfamate of Formula X is used at an amount of about 0.1 to 2.0 moles. As the base catalyst, sodium hydroxide, potassium hydroxide, lithium hydroxide, potassium carbonate, or potassium cyanide is used at an amount of 0.1 to 1.0 equivalent at a temperature of 0 to 30°C. Useful solvents include methylalcohol, ethylalcohol and propylalcohol. Preferred is melhylalcohol when considering the reaction yield and the removal convenience after completion of the reaction. As in the first method for the preparation of the compound of Formula I, the base catalyst remained in the reaction mixture is required to be inactivated after completion of the transesterification. For this, 1N hydrochloric acid solution or saturated ammonium chloride solution is preferably used.

There is a subsequent procedure in Reaction Scheme XIII: 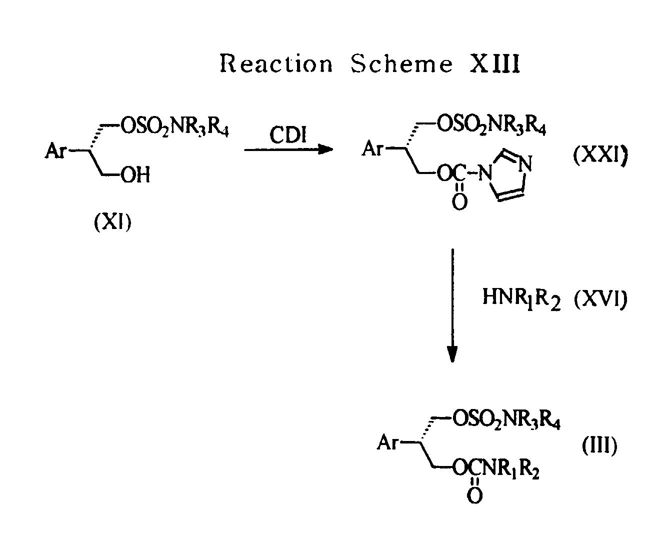 wherein Ar, R₁, R₂, R₃ and R₄ each are as defined above.

In Reaction Scheme XIII, the (S)-2-aryl-1,3-propandiol monosulfamate of Formula XI is reacted with carbonyl diimidazole in dichloromethane solvent to give the (S)-3-imidazolyl carbonyloxy-2-arylpropanol sulfamate which is, without purification, with the substituted amine of Formula XVI, to prepare the N,N'-substituted carbamoyl-2-aryl propanol sulfamate of Formula IIl, the objective compound.

About 0.1 to 2.0 moles of the compound of Formula XI is preferably reacted with 1.1 to 2.5 equivalents of carbonyl diimidazole when considering yield and economical aspects. It is preferable that the former reaction of Reaction Scheme XIII is carried out at a temperature of -5 to 40°C. For example, if the reaction is performed at a temperature less than -5°C, the reaction is very slow. On the other hand, if it is carried out at a temperature higher than 40°C, the yield decreases by side reactions. Useful solvents for this reaction include low hydrocarbon halide solvents, such as methylene chloride and chloroform, ethereal solvents, such as ethyl ether and tetrahydrofuran, and acetonitrile with preference to methylene chloride, chloroform and tetrahydrofuran.

In the preparation reaction of the compound of Formula III from the compound of Formula XXI, the substituted amine is used at an amount of 1.0 to 5.0 equivalents at a temperature ranging from 0 to 30°C in a solvent such as tetrahydrofuran and methylene chloride.

Alternatively, the compound of Formula III can be prepared by using (S)-3-acetoxy-2-aryl propanol of Formula XXII as the starting material, in accordance with the present invention, as shown in Reaction Scheme XIV: 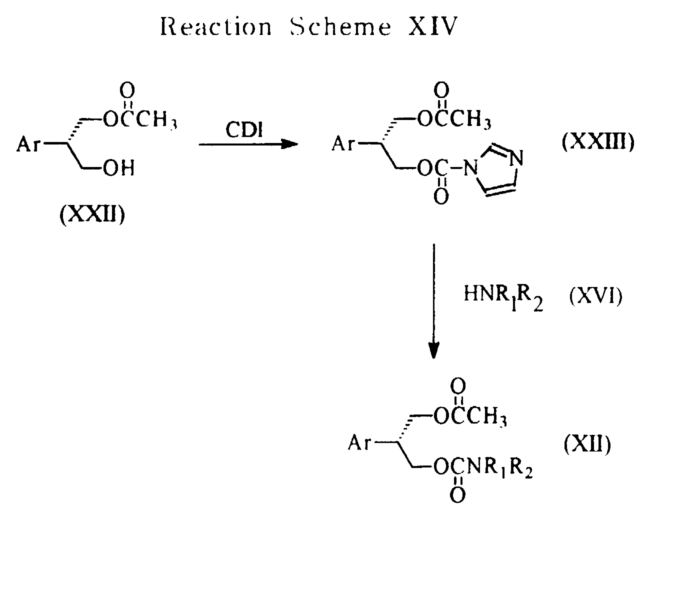 wherein Ar, R₁ and R₂ each are as defined above.

The (S)-3-acetoxy-2-aryl propanol of Formula XXII is reacted with carbonyl diimidazole in dichloromethane solution to give the (R)-3-imidazolyl carbonyloxy-2-aryl propanol acetate of Formula XXIII, which is, without purification, reacted with the substituted amine of Formula XVI, to produce the (R)-3-N-substituted carbamoyl-2-aryl propanol acetate of Formula XII.

The compound of Formula XXII is reacted preferably at an amount of about 0.1 to 2.0 moles with 1.1 to 2.5 equivalents of carbonyl diimidazole when considering yield and economical aspects. It is preferable that the former reaction of Reaction Scheme XIV is carried out at a temperature of -5 to 40°C. For example, if the reaction is performed at a temperature less than -5°C, the reaction is very slow. On the other hand, if it is carried out at a temperature higher than 40°C, the yield decreases by side reactions. Useful solvents in this reaction include low hydrocarbon halide solvents, such as methylene chloride and chloroform, ethereal solvents, such as ethyl ether and tetrahydrofuran, and acetonitrile with preference to methylene chloride, chloroform and tetrahydrofuran.

The preparation reaction of the compound of Formula XII from the compound of Formula XXIII is carried out with 1.0 to 5.0 equivalents of the substituted amine at a temperature ranging from 0 to 30°C in a solvent such as tetrahydrofuran and methylene chloride.

There is a subsequent procedure in Reaction Scheme XV: 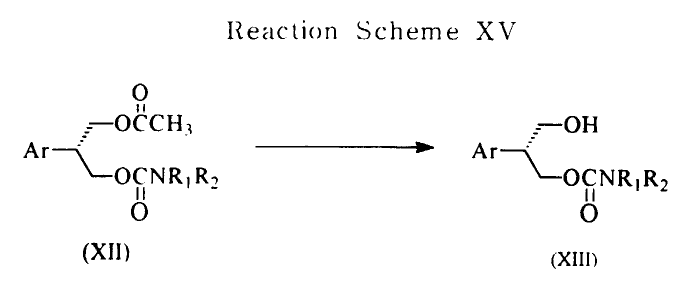 wherein Ar, R₁ and R₂ each are as defined above.

The (R)-3-N-substituted carbamoyl-2-aryl propanol acetate of Formula XII is subjected to transesterification to obtain the (R)-3-N-substituted carbamoyl-2-aryl propanol of Formula XIII. This reaction is executed in an alcohol solvent in the presence of a base catalyst.

In the transesterification, the (R)-3-N-substituted carbamoyl-2-aryl propanol acetate of Formula XII is used at an amount of 0.1 to 2.0 moles. As the base catalyst, sodium hydroxide, potassium hydroxide, lithium hydroxide, potassium carbonate, or potassium cyanide is used at an amount of 0.1 to 1.0 equivalent at a temperature of 0 to 30 °C. Useful solvents include methylalcohol, ethylalcohol and propylalcohol. Preferred is methylalcohol when considering the reaction yield and the removal convenience after completion of the reaction. As in the first and the second methods for the preparation of the compound of Formula I, it is needed to inactivate the used base catalyst after the transesterification. For the inactivation of the base catalyst, 1N hydrochloric acid solution or saturated ammonium chloride solution is preferably used.

Next, the (R)-3-N-substituted carbamoyl-2-aryl propanol of Formula XIII is reacted with sulfamoyl chloride in the presence of a base catalyst, to prepare the objective compound of Formula III. This reaction is depicted in Reaction Scheme XVI: 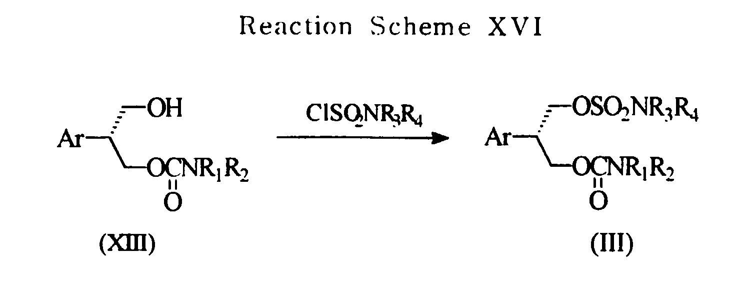 wherein Ar, R₁, R₂, R₃, and R₄ each are as defined above.

The (R)-3-N-substituted carbamoyl-2-aryl propanol represented by Formula XIII is used at an amount of 0.1 to 2.0 moles while sulfamoyl chloride at an amount of about 1.0 to 3.0 equivalents. As the base catalyst, triethylamine, pyridine, antipyrin, or diisopropylethylamine is employed. When considering reaction progress, convenience in after-treatment and economical aspects, it is preferable that the base catalyst is used at an amount ranging from 2.0 to 4.0 equivalents. Available reaction solvents include amide solvents, such as dimethylformamide, ethereal solvents, such as ethyl ether and tetrahydrofuran, and acetonitrile with preference to tetrahydrofuran and acetonitrile. It is preferable that this reaction is carried out at a temperature of -10 to 40°C. For example, if the reaction temperature is maintained below - 10°C, the reaction is very slow. On the other hand, if the reaction is carried out at a temperature higher than 40°C, the yield decreases by the production of unknown by-products.

A better understanding of the present invention may be obtained in light of following examples which are set forth toillustrate, but are not to be construed to limit, the present invention.

### EXAMPLE 1

### Preparation of 3-Carbamoyl-2-phenyl propanol acetate

A well dried 1,000 ml flask equipped with a thermometer was purged with nitrogen gas, to completely take off moisture and air from the inside of the flask. This purging was continued for 30 min, after which 400 ml of purified methylene chloride was poured into the flask. 50 g of 3-acetoxy-2-phenyl propanol was well dissolved in the solvent and made to be homogeneous by stirring for 5 min. While being maintained at 0°C, the homogeneous solution was slowly added with 49.8 g of carbonyl diimidazole. After the starting material completely disappeared as measured by thin layer chromatography, 43.8 ml of aqueous ammonia was slowly added to the reaction solution in order to progress the reaction.

To determine when the reaction would be terminated, the reaction was under double monitoring of thin layer chromatography and liquid chromatography. Roughly, it took about 1 hour to finish the reaction.

After completion of reaction, 400 ml of distilled water was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (2:1) as a mobile phase, to obtain 58.0 g of 3-carbamoyl-2-phenyl propanol acetate as a white solid.
Yield 95%
H-NMR(CDCl₃, 200 MHz), ppm (δ): 1.98(3H,s),
3.28(1H,q), 4.28(4H,d), 5.21(2H,br), 7.27(5H,m)

### EXAMPLE 2

### Preparation of 3-N-melhylcarbamoyl-2-phenyl propanol acetate

Except for using methylamine instead of aqueous ammonia, the same procedure with that of Example 1 was repeated.

A concentrated reaction mixture thus obtained was separated by column chromatography using a mixture of ethyl acetate/n-hexane (2:1) as a mobile phase, to obtain 58.9 g of 3-N-methylcarbamoyl-2-phenyl propanol acetate as a colorless liquid.
Yield 91%
H-NMR(CDCl₃, 200 MHz), ppm (δ): 1.95(3H,s), 2.75(3H,d), 3.28(1H,q), 4.21(4H,d), 5.15(2H,br), 7.27(5H,m)

### EXAMPLE 3

### Preparation of 3-N,N'-dimethylcarbamoyl-2-phenyl propanol acetate

Except for using dimethylamine instead of aqueous ammonia, the same procedure with that of Example 1 was repeated.

A concentrated reaction mixture thus obtained was separated by column chromatography using a mixture of ethyl acetate/n-hexane (2:1) as a mobile phase, to obtain 63.5 g of 3-N,N'-dimethylcarbamoyl-2-phenyl propanol acetate as a colorless liquid.
Yield 93%
H-NMR(CDCl₃, 200 MHz), ppm (δ): 1.97(3H,s),
2.78(6H,d), 3.34(1H,q), 4.17(2H,d), 4.39(2H,d), 7.32(5H,m)

### EXAMPLE 4

### Preparation of 3-N-isopropyl carbamoyl-2-phenyl propanol acetate

Except for using isopropylamine instead of aqueous ammonia, the same procedure with that of Example 1 was repeated.

A concentrated reaction mixture thus obtained was separated by column chromatography using a mixture of ethyl acetate/n-hexane (2:1) as a mobile phase, to obtain 64.7 g of 3-N-isopropylcarbamoyl-2-phenyl propanol acetate as a colorless liquid.
Yield 90%
H-NMR(CDCl₃, 200 MHz), ppm (δ): 1.11(6H,s),
1.90(3H,s), 3.35(1H,m), 3.71(1H,br), 4.43(4H,d), 4.64(1H,br), 7.35(5H,m)

### EXAMPLE 5

### Preparation of 3-N-cyclopropyl carbamoyl-2-phenyl propanol acetate

Except for using cyclopropyl amine instead of aqueous ammonia, the same procedure with that of Example 1 was repeated.

A concentrated reaction mixture thus obtained was separated by column chromatography using a mixture of ethyl acetate/n-hexane (2:1) as a mobile phase, to obtain 67.8 g of 3-N-cyclopropyl carbamoyl-2-phenyl propanol acetate as a colorless liquid.
Yield 95%
H-NMR(CDCl₃, 200 MHz), ppm (δ): 0.45-0.81(6H,m), 2.01(3H,s), 2.52(1H,br), 3.37(1H,q), 4.45(4H,d), 4.87(1H,br), 7.32(5H,m)

### EXAMPLE 6

### Preparation of 3-N-morphoryl carbamoyl-2-phenyl propanol acetate

Except for using morphorine instead of aqueous ammonia, the same procedure with that of Example 1 was repeated.

A concentrated reaction mixture thus obtained was separated by column chromatography using a mixture of ethyl acetate/n-hexane (2:1) as a mobile phase, to obtain 72.8 g of 3-N-morphorylcarbamoyl-2-phenyl propanol acetate as a colorless liquid.
Yield 92%
H-NMR(CDCl₃, 200 MHz), ppm (δ): 2.00(3H,s),
3.25-3.73(9H,br), 4.31(4H,d), 7.27(5H,m)

### EXAMPLE 7

### Preparation of 3-N-methylcarbamoyl-2-(o-chlorophenyl) propanol acetate

A well dried 1,000 ml flask equipped with a thermometer was purged with nitrogen gas, to completely take off moisture and air from the inside of the flask. This purging was continued for 30 min, after which 400 ml of purified methylene chloride was poured into the flask. 58.9 g of 3-acetoxy-2-(o-chlorophenyl)propanol was well dissolved in the solvent and made to be homogeneous by stirring for 5 min. While being maintained at 0°C, the homogeneous solution was slowly added with 49.8 g of carbonyl diimidazole. After the starting material completely disappeared as measured by thin layer chromatography, 60 ml of an aqueous methylamine solution was slowly added to the reaction solution in order to progress the reaction.

To determine when the reaction would be terminated, the reaction was under double monitoring of thin layer chromatography and liquid chromatography. Roughly, it took about 1 hour to finish the reaction.

After completion of reaction, 400 ml of distilled water was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (2:1) as a mobile phase, to obtain 68.5 g of 3-N-methylcarbamoyl-2-(o-chlorophenyl) propanol acetate as a colorless liquid.
Yield 93%
H-NMR(CDCl₃, 200 MHz), ppm (δ): 2.05(3H,s), 2.82(d,3H), 3.32(1H,q), 4.15-4.27(4H,m), 5.32(1H,br), 7.23-7.45(4H,m)

### EXAMPLE 8

### Preparation of 3-Carbamoyl-2-Phenyl Propanol

A well dried 1.000 ml flask equipped with a thermometer was purged with nitrogen gas, to completely lake off moisture and air from the inside of the flask. This purging was continued for 30 min, after which 500 ml of methanol was poured into the flask. 71 g of 3-carbamoyl-2-phenyl propanol acetate prepared in Example 1 was well dissolved in the solvent and made to be homogeneous by stirring for 30 min. While being maintained at 0°C, the homogeneous solution was slowly added with 3.3 g of potassium cyanide. The resulting reaction mixture was heated to room temperature with stirring. To determine when the reaction would be terminated, the reaction was under double monitoring of thin layer chromatography and liquid chromatography. Roughly, it took about 2 hours to finish the reaction.

After completion of the reaction, 300 ml of saturated ammonium chloride solution was added. Then, using a rotary evaporator, unreacted methanol was completely removed from the reaction solution. 200 ml of distilled water and 500 ml of ethyl acetate were added for solvent extraction. The organic layer thus obtained was dried over anhydrous magnesium sulfate and distilled off the solvent by a rotary evaporator, to give a yellow liquid.

This liquid obtained was added with 300 ml of toluene and stored in a refrigerator for 24 hrs, to produce 51.5 g of 3-carbamoyl-2-phenyl propanol as a white solid.
Yield 88%
H-NMR(CDCl₃, 200 MHz), ppm (δ): 2.58(1H,t),
3.11(1H,q), 3.82(2H,d), 4.39(2H,d), 4.91(2H,br), 7.27(5H,m)

### EXAMPLE 9

### Preparation of 3-N-methylcarbamoyl-2-phenyl propanol

Except for using 3-N-methylcarbamoyl-2-phenyl propanol acetate, instead of 3-carbamoyl-2-phenyl propanol acetate, as the starting material, the same procedure with that of Example 8 was repeated.

A concentrated reaction mixture thus obtained was separated by column chromatography using a mixture of ethyl acetate/n-hexane (3:1) as a mobile phase, to obtain 58.3 g of 3-N-methylcarbamoyl-2-phenyl propanol as a white solid.
Yield 93%
H-NMR(DMSO, 200 MHz), ppm (δ): 2.52(3H,d), 3.01(1H,q), 3.61(2H,t), 4.18(2H,m), 4.75(1H,t), 6.89(1H,d), 7.28(5H,m)

### EXAMPLE 10

### Preparation of 3-N,N'-Dimethylcarbamoyl-2-phenyl propanol

Except for using 3-N,N'-dimethylcarbamoyl-2-phenyl propanol acetate, instead of 3-carbamoyl-2-phenyl propanol acetate, as the starting material, the same procedure with that of Example 8 was repeated.

A concentrated reaction mixture thus obtained was separated by column chromatography using a mixture of ethyl acetate/n-hexane (1:1) as a mobile phase, to obtain 60.9 g of 3-N,N'-dimethylcarbamoyl-2-phenyl propanol as a colorless liquid.
Yield 91%
H-NMR(DMSO, 200 MHz), ppm (δ): 2.61(6H,d), 3.10(1H,q), 3.73(2H,d), 4.12(2H,d), 4.74(1H,t), 7.34(5H,m)

### EXAMPLE 11

### Preparation of 3-N-Isopropyl carbamoyl-2-phenyl propanol

Except for using 3-N-isopropyl carbamoyl-2-phenyl propanol acetate, instead of 3-carbamoyl-2-phenyl propanol acetate, as the starting material, the same procedure with that of Example 8 was repeated.

A concentrated reaction mixture thus obtained was separated by column chromatography using a mixture of ethyl acetate/n-hexane (1:1) as a mobile phase, to obtain 63.3 g of 3-N-isopropyl carbamoyl-2-phenyl propanol as a white solid.
Yield 89%
H-NMR(DMSO, 200 MHz), ppm (δ): 1.00(6H,d), 2.52(1H,d), 2.95(1H,t), 3.62(2H,d), 4.18(2H,m), 4.74(1H,t), 6.91(1H,d), 7.28(5H,m)

### EXAMPLE 12

### Preparation of 3-N-Cyclopropyl carbamoyl-2-phenyl propanol

Except for using 3-N-cyclopropyl carbamoyl-2-phenyl propanol acetate, instead of 3-carbamoyl-2-phenyl propanol acetate, as the starting material, the same procedure with that of Example 8 was repeated.

A concentrated reaction mixture thus obtained was separated by column chromatography using a mixture of ethyl acetate/n-hexane (1:1) as a mobile phase, to obtain 65.6 g of 3-N-cyclopropyl carbamoyl-2-phenyl propanol acetate as a white solid.
Yield 93%
H-NMR(DMSO, 200 MHz), ppm (δ): 0.35-0.55(4H,m), 2.52(1H,s), 3.01(1H,t), 3.61(2H,d), 4.25(2H,m), 4.76(1H,t), 7.14(1H,d), 7.28(5H,m)

### EXAMPLE 13

### Preparation of 3-N-Morphoryl carbamoyl-2-phenyl propanol

Except for using 3-N-morphoryl carbamoyl-2-phenyl propanol acetate, instead of 3-carbamoyl-2-phenyl propanol acetate, as the starting material, the same procedure with that of Example 8 was repeated.

A concentrated reaction mixture thus obtained was separated by column chromatography using a mixture of ethyl acetate/n-hexane (3:1) as a mobile phase, to obtain 71.4 g of 3-N-morphorylcarbamoyl-2-phenyl propanol as a white solid.
Yield 90%
H-NMR(DMSO, 200 MHz), ppm(δ): 2.82(1H,br),
3.15(1H,q), 3.27-3.60(8H,br), 3.79(2H,d), 4.35(2H,d), 7.25(5H,m)

### EXAMPLE 14

### Preparation of 3-N-Methylcarbamoyl-2-(o-chlorophenyl) propanol

A well dried 1,000 ml flask equipped with a thermometer was purged with nitrogen gas, to completely take off moisture and air from the inside of the flask. This purging was continued for 30 min, after which 500 ml of methanol was poured into the flask. 85.7 g of 3-N-methylcarbamoyl-2-(o-chlorophenyl) propanol acetate prepared in Example 7 was well dissolved in the solvent and made to he homogeneous by stirring for 30 min. While being maintained at 0°C, the homogeneous solution was slowly added with 3.3 g of potassium cyanide. The resulting reaction mixture was heated to room temperature with stirring. To determine when the reaction would be terminated, the reaction was under double monitoring of thin layer chromatography and liquid chromatography. Roughly, it took about 2 hours to finish the reaction.

After completion of the reaction, 300 ml of saturated ammonium chloride solution was added. Then, using a rotary evaporator, unreacted methanol was completely removed from the reaction solution. 200 ml of distilled water and 500 ml of ethyl acetate were added for solvent extraction. The organic layer thus obtained was dried over anhydrous magnesium sulfate and distilled off the solvent by a rotary evaporator, to give a yellow liquid.

This liquid obtained was added with 500 ml of toluene and stored in a refrigerator for 24 hrs, to produce 65.8 g of 3-N-methylcarbamoyl-2-(o-chlorophenyl) propanol as a white solid.
Yield 90%
H-NMR(CDCl₃, 200 MHz), ppm (δ): 2.48(3H,d), 2.56(1H,t), 3.13(1H,q), 3.87-4.12(4H,br), 5.60(1H,br), 7.25-7.42(4H,m)

### EXAMPLE 15

### Preparation of 3-Carbamoyl-2-phenyl propanol sulfamate

A well dried 2,000 ml flask equipped with a thermometer was purged with nitrogen gas, to completely take off moisture and air from the inside of the flask. This purging was continued for 30 min, after which 1,200 ml of purified acetonitrile was poured into the flask. 97.5 g of 3-carbamoyl-2-phenyl propanol prepared in Example 8 and 121.3 ml of pyridine were well dissolved in the solvent and made to be homogeneous by stirring for 30 min. While being maintained at 0°C, the homogeneous solution was slowly added with 115.5 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, unreacted acetonitrile was completely removed from the reaction solution using a rotary evaporator. Then, 600 ml of distilled water and 600 ml of ethyl acetate were added for solvent extraction. The organic layer thus obtained was dried over anhydrous magnesium sulfate and distilled off the solvent by a rotary evaporator, to give a yellow liquid.

This liquid obtained was added with 500 ml of ethyl ether and stored in a refrigerator for 24 hrs, to produce 116.5 g of 3-carbamoyl-2-phenyl propanol sulfamates a white solid.
Yield 85%
H-NMR(DMSO, 200 MHz), ppm (δ): 3.35(1H,q), 4.16(2H,d), 4.34(2H,d), 6.57(2H,br), 7.39(5H,s), 7.58(2H,s)

### EXAMPLE 16

### Preparation of 3-N-Melhylcarbamoyl-2-phenyl propanol sulfamate

Except for using 3-N-methylcarbamoyl-2-phenyl propanol, instead of 3-carbamoyl-2-phenyl propanol, as the starting material, the same procedure with that of Example 15 was repeated.

A concentrated reaction mixture thus obtained was added with 500 ml of ethyl ether and stored in a refrigerator for 24 hrs, to produce 126.7 g of 3-N-methylcarbamoyl-2-phenyl propanol sulfamate as a white solid.
Yield 88%
H-NMR(DMSO, 200 MHz), ppm(δ): 2.54(3H,d), 3.35(1H,q), 4.29(4H,m), 6.98(1H,br), 7.29(5H,s), 7.53(2H,s)

### EXAMPLE 17

### Preparation of 3-N,N'-Dimethylcarbamoyl-2-phenyl propanol sulfamate

Except for using 3-N,N'-dimethylcarbamoyl-2-phenyl propanol, instead of 3-carbamoyl-2-phenyl propanol, as the starting material, the same procedure with that of Example 15 was repeated.

A concentrated reaction mixture thus obtained was added with 500 ml of ethyl ether and stored in a refrigerator for 24 hrs, to produce 125.3 g of 3-N,N'-dimethylcarbamoyl-2-phenyl propanol sulfamate as a white solid.
Yield 83%
H-NMR(CDCl₃, 200 MHz), ppm(δ): 2.84(6H,d), 3.38(1H,q), 4.39(4H,m, 4.93(2H,s), 7.21(5H,d)

### EXAMPLE 18

### Preparation of 3-N-Isopropyl carbamoyl-2-phenyl propanol sulfamate

Except for using 3-N-isopropyl carbamoyl-2-phenyl propanol, instead of 3-carbamoyl-2-phenyl propanol, as the starting material, the same procedure with that of Example 15 was repeated.

A concentrated reaction mixture thus obtained was added with 500 ml of ethylether and stored in a refrigerator for 24 hrs, to produce 134.3 g of 3-N-isopropyl carbamoyl-2-phenyl propanol sulfamate as a white solid.
Yield 85%
H-NMR(DMSO, 200 MHz), ppm(δ): 1.01(6H,m), 3.32(1H,m), 3.55(1H,q), 4.18(2H,d), 4.29(2H,d), 6.95(1H,d), 7.34(5H,s), 7.56(2H,s)

### EXAMPLE 19

### Preparation of 3-N-Cyclopropyl carbamoyl-2-phenyl propanol sulfamate

Except for using 3-N-cyclopropyl carbamoyl-2-phenyl propanol, instead of 3-carbamoyl-2-phenyl propanol, as the starting material, the same procedure with that of Example 15 was repeated.

A concentrated reaction mixture thus obtained was added with 500 ml of ethylether and stored in a refrigerator for 24 hrs, to produce 135.0 g of 3-N-cyclopropyl carbamoyl-2-phenyl propanol sulfamate as a white solid.
Yield 86%
H-NMR(DMSO, 200MHz) ppm(δ): 0.35-0.58(4H,m), 2.51(1H,s), 3.35(1H,q), 4.15-4.36(4H,m), 7.15(1H,br), 7.29(5H,s), 7.52(2H,s)

### EXAMPLE 20

### Preparation of 3-N-Morphoryl carbamoyl-2-phenyl propanol sulfamate

Except for using 3-N-morphoryl carbamoyl-2-phenyl propanol, instead of 3-carbamoyl-2-phenyl propanol, as the starting material, the same procedure with that of Example 15 was repeated.

The liquid thus obtained was added with 500 ml of ethylether and stored in a refrigerator for 24 hrs, to produce 163.6 g of 3-N-morphorylcarbamoyl-2-phenyl propanol sulfamate as a white solid.
Yield 95%
H-NMR(CDCl₃, 200MHz) ppm(δ): 3.05(1H,q) 3.20-3.50(8H,br), 4.15(4H,m), 4.43(2H,s), 7.13(5H,m)

### EXAMPLE 21

### Preparation of 3-N-Methylcarbamoyl-2-(o-chlorophenyl) propanol sulfamate

A well dried 2,000 ml flask equipped with a thermometer was purged with nitrogen gas, to completely take off moisture and air from the inside of the flask. This purging was continued for 30 min, after which 1,200 ml of purified acetonitrile was poured into the flask. 121.8 g of 3-N-methylcarbamoyl-2-(o-chlorophenyl) propanol prepared in Example 14 and 121.3 ml of pyridine were well dissolved in the solvent and made to be homogeneous by stirring for 30 min. While being maintained at 0°C, the homogeneous solution was slowly added with 113.5 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, acetonitrile was completely removed from the reaction mixture by using a rotary evaporator. 600 ml of distilled water and 600 ml of ethyl acetate were added for solvent extraction. The organic layer thus obtained was dried over anhydrous magnesium sulfate and distilled off the solvent by a rotary evaporator, to give a yellow liquid.

This liquid obtained was added with 500 ml to of ethylether and stored in a refrigerator for 24 hrs, to produce 143.6 g of 3-N-methylcarbamoyl-2-(o-chlorophenyl) propanol sulfamate as a white solid.
Yield 89%
H-NMR(DMSO, 200 MHz), ppm (δ): 2.53(3H,d), 3.32(1H,q), 4.33(4H,m), 6.53(1H,br), 7.35(4H,m), 7.62(2H,s)

### EXAMPLE 22

### Preparation of 3-Acetoxy-2-phenyl-1,3-propandiol sulfamate

A well dried 1,000 ml flask equipped with a thermometer was purged with nitrogen gas, to completely take off moisture and air from the inside of the flask. This purging was continued for 30 min, after which 300 ml of purified acetonitrile was poured into the flask. 50 g of 3-acetoxy-2-phenyl propanol and 61 ml of pyridine were well dissolved in the solvent and made to be homogeneous by stirring for 30 min. While being maintained at 0°C, the homogeneous solution was slowly added with 59.6 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, acetonitrile was completely removed from the reaction solution using a rotary evaporator. Then, 300 ml of distilled water and 300 ml of ethyl acetate were added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, to produce 60.1 g of 3-acetoxy-2-phenyl-1,3-propandiol sulfamate as a white solid.
Yield 87%
H-NMR(DMSO, 200MHz), ppm(δ): 1.95(3H,s), 3.36(1H,q), 4.28(4H,d), 7.28(5H,s), 7.53(2H,s)

### EXAMPLE 23

### Preparation of 2-Phenyl-1,3-propandiol monosulfamate

A well dried 1,000 ml flask equipped with a thermometer was purged with nitrogen gas, to completely take off moisture and air from the inside of the flask. This purging was continued for 30 min, after which 500 ml of methanol was poured into the flask. 27.3 g of 3-acetoxy-2-phenyl-1,3-propandiol sulfamate prepared in Example 22 was well dissolved in the solvent and made to be homogeneous by stirring for 30 min. While being maintained at 0°C, the homogeneous solution was slowly added with 3.3 g of potassium cyanide. The resulting reaction mixture was heated to room temperature with stirring. To determine when the reaction would be terminated, the reaction was under double monitoring of thin layer chromatography and liquid chromatography. Roughly, it tools about 1 hours to finish the reaction.

After completion of the reaction, 50 ml of saturated ammonium chloride solution was added. Then, using a rotary evaporator, unreacted methanol was completely removed from the reaction solution. 200 ml of distilled water and 200 ml of ethyl acetate were added for solvent extraction. The organic layer thus obtained was dried over anhydrous magnesium sulfate and distilled off the solvent by a rotary evaporator, to give a yellow solid.

This solid was washed with 50 ml of methylene chloride and purified to produce 19 g of 2-phenyl-1,3-propandiol monosulfamate.
Yield 81%
H-NMR(DMSO, 200MHz), ppm(δ): 3.12(1H,q), 3.61(2H,d), 4.24(2H,m), 4.86(1H,t), 7.15(5H,m), 7.46(2H,s)

### EXAMPLE 24

### Preparation of 3-N-Methylcarbamoyl-2-phenyl propanol sulfamate

A well dried 1,000 ml flask equipped with a thermometer was purged with nitrogen gas, to completely take off moisture and air from the inside of the flask. This purging was continued for 30 min, after which 300 ml of purified methylene chloride was poured into the flask. 23.3 g of 2-phenyl-1,3-propandiol monosulfamate prepared in Example 23 was well dissolved in the solvent and made to he homogeneous by stirring for 30 min. While being maintained at 0°C, the homogeneous solution was slowly added with 19.4 g of carbonyl diimidazole. The resulting reaction mixture was slowly heated to room temperature with stirring. To determine when the reaction would be terminated, the reaction was under double monitoring of thin layer chromatography and liquid chromatography. Roughly, it took about 15 m in to finish the reaction. After the starting material completely disappeared, 47 ml of methyl amine aqueous solution was slowly introduced through a syringe with stirring at room temperature. It took about 2 hours to completely terminate this reaction.

Then, using a rotary evaporator, the solvent remaining was completely removed from the reaction solution. 200 ml of distilled water and 200 ml of ethyl acetate were added for solvent extraction. The organic layer thus obtained was dried over anhydrous magnesium sulfate and distilled off the solvent by a rotary evaporator, to give a yellow liquid.

This solid was washed with 150 ml of ethyl ether and stored in a refrigerator for 24 hrs, to produce 24.4 g of 3-N-methylcarbamoyl-2-phenyl propanol sulfamate.
Yield 85%
H-NMR(DMSO, 200MHz), ppm(δ): 2.54(3H, d), 3.35(1H,q), 4.29(4H,m), 6.89(1H,br), 7.29(5H,s), 7.53(2H,s)

### EXAMPLE 25

### Preparation of 3-Carbamoyl-2-phenyl propanol sulfamate

Except for using aqueous ammonia instead of methylamine, the same procedure with that of Example 24 was repeated.

A liquid obtained was added with 150 ml of ethyl ether and stored in a refrigerator for 24 hrs to produce 27.0 g of 3-carbamoyl-2-phenyl propanol sulfamate as a white solid.
Yield 80%
H-NMR(DMSO, 200 MHz), ppm (δ): 3.35(1H,q), 4.16(2H,d), 4.34(2H,d), 6.57(2H,br), 7.39(5H,s), 7.58(2H,s), 1.98(3H,s), 3.28(1H,q)

### EXAMPLE 26

### Preparation of 3-N,N'-Dimethylcarbamoyl-2-phenyl propanol sulfamate

Except for using dimethylamine instead of methylamine, the same procedure with that of Example 24 was repeated.

A liquid obtained was added with 150 ml of ethylether and stored in a refrigerator for 24 hrs, to produce 27.0 g of 3-N,N'-dimethylcarbamoyl-2-phenyl to propanol sulfamate as a white solid.
Yield 80%
H-NMR(DMSO, 200MHz), ppm (δ): 2.84(6H,d), 3.38(1H,q), 4.39(4H,m), 4.93(2H,s), 7.21(5H,d)

### EXAMPLE 27

### Preparation of 3-N-Isopropyl carbamoyl-2-phenyl propanol sulfamate

Except for using isopropyl amine instead of methyl amine the same procedure with that of Example 24 was repeated.

A liquid obtained was added with 150 ml of ethylether and stored in a refrigerator for 24 hrs, to produce 26.0 g of 3-N-isopropyl carbamoyl-2-phenyl propanol sulfamate.
Yield 82%
H-NMR(DMSO, 200MHz, ppm(δ): 1.01(6H,m), 3.32(1H,m), 3.55(1H,q), 4.18(2H,d), 4.29(2H,d), 6.95(1H,d), 7.34(5H,s)

### EXAMPLE 28

### Preparation of 3 N-Cyclopropyl carbamoyl-2-phenyl propanol sulfamate

Except for using cyclopropyl amine instead of methyl amine, the same procedure with that of Example 24 was repeated.

A liquid obtained was added with 150 ml of ethylether and stored in a refrigerator for 24 hrs, to produce 27.6 g of 3-N-cyclopropyl carbamoyl-2-phenyl propanol sulfamale.
Yield 88%
H-NMR(DMSO, 200MHz) ppm(δ): 0.35-0.58(4H,m), 2.51(1H,s), 3.35(1H,q), 4.16-4.36(4H,m), 7.15(1H,br), 7.29(5H,s)

### EXAMPLE 29

### Preparation of 3-N-Morphoryl carbamoyl-2-phenyl propanol sulfamate

Except for using morphorine instead of methyl amine, the same procedure with that of Example 24 was repeated.

A liquid thus obtained was added with 500 ml of ethylether and stored in a refrigerator for 24 hrs, to produce 32 g of 3-N-morphorylcarbamoyl-2-phenyl propanol sulfamate.
Yield 93%
H-NMR(DMSO, 200MHz) ppm(δ): 3.05(1H,q) 3.20-3.50(8H,br), 4.15(4H,m), 4.43(2H,s), 7.13(5H,m)

### EXAMPLE 30

### Preparation of 3-N-Phenyl carbamoyl-2-phenyl propanol sulfamate

A well dried 1,000 ml flask equipped with a thermometer and a condenser was purged with nitrogen gas, to completely take off moisture and air from the inside of the flask. This purging was continued for 30 min, after which 300 ml of purified dichloromethane was poured into the flask. 23.3 g of 2-phenyl-1,3-propandiol monosulfamate prepared in Example 20 was well dissolved in the solvent and made to be homogeneous by stirring for 30 min. While being maintained at room temperature, the homogeneous solution was slowly added with 14.3 g of phenylisocyanate. The resulting solution was slowly heated into 40°C with stirring, in order to progress the reaction.

To determine when the reaction would be terminated, the reaction was under double monitoring of thin layer chromatography and liquid chromatography. Roughly, it took about 6 hours to finish the reaction.

After completion of reaction, dichloromethane remaining was removed from the solution by use of a rotary evaporator. 100 ml of distilled water and 100 ml of ethyl acetate was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, to obtain 24.5 g of 3-N-phenyl carbamoyl-2-phenyl propanol sulfamate.
Yield 70%
H-NMR(CDCl₃, 200MHz), ppm(δ): 3.46(1H,m), 4.41 (4H,m), 5.05(2H,br), 7.05(1H,br), 7.31(10H,m)

### EXAMPLE 31

### Preparation of (S)-3-Carbamoyl-2-phenyl propanol acetate

A well dried 1,000 ml flask equipped with a thermometer was purged with nitrogen gas, to completely take off moisture and air from the inside of the flask. This purging was continued for 30 min, after which 500 ml of purified methylene chloride was poured into the flask. 50 g of (R)-3-acetoxy-2-phenyl propanol was well dissolved in the solvent and made to be homogeneous by stirring for 5 min. While being maintained at 0°C, the homogeneous solution was slowly added with 49.8 g of carbonyl diimidazole. After the starting material completely disappeared as measured by thin layer chromatography, 43.8 ml of aqueous ammonia was slowly added to the reaction solution in order to progress the reaction.

To determine when the reaction would be terminated, the reaction was under double monitoring of thin layer chromatography and liquid chromatography. Roughly, it took about 2 hours to finish the reaction.

After completion of reaction, 500 ml of distilled water was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, in obtain 56.2 g of (S)-3-carbamoyl-2-phenyl propanol acetate.
Yield 92%
H-NMR(DMSO, 200MHz), ppm(δ): 1.98(3H,s), 3.28(1H,q), 4.28(4H,d), 5.21(2H,br), 7.27(5H,m)

### EXAMPLE 32

### Preparation of (S)-3-N-methylcarbamoyl-2-phenyl propanol acetate

Except for using methylamine instead of aqueous ammonia, the same procedure with that of Example 31 was repeated.

A concentrated reaction mixture thus obtained was separated by column chromatography using a mixture of ethyl acetate/n-hexane (1:1) as a mobile phase, to obtain 58.2 g of (S)-3-N-methylcarbamoyl-2-phenyl propanol acetate as a colorless liquid.
Yield 90%
H-NMR(CDCl₃, 200MHz), ppm(δ): 1.95(3H,s), 2.75(3H,d), 3.28(1H,q), 4.21(4H,d), 5.15(1H,br), 7.27(5H,m)

### EXAMPLE 33

### Preparation of (S)-3-N,N'-dimethylcarbamoyl-2-phenyl propanol acetate

Except for using dimethylamine instead of aqueous ammonia, the same procedure with that of Example 31 was repeated.

A concentrated reaction mixture thus obtained was separated by column chromatography using a mixture of ethyl acetate/n-hexane (1:1) as a mobile phase, to obtain 62.8 g of (S)-3-N,N'-dimethylcarbamoyl-2-phenyl propanol acetate as a colorless liquid.
Yield 92%
H-NMR(CDCl₃, 200MHz), ppm(δ):1.97(3H,s), 2.78(6H,d), 3.34(1H,q), 4.17(2H,d), 4.39(2H,d), 7.32(5H,m)

### EXAMPLE 34

### Preparation of (S)-3-N-isopropyl carbamoyl-2-phenyl propanol acetate

Except for using isopropylamine instead of aqueous ammonia, the same procedure with that of Example 31 was repeated.

A concentrated reaction mixture thus obtained was separated by column chromatography using a mixture of ethyl acetate/n-hexane (1:1) as a mobile phase, to obtain 66.9 g of (S)-3-N-isopropylcarbamoyl-2-phenyl propanol acetate as a colorless liquid.
Yield 93%
H-NMR(CDCl₃, 200MHz), ppm(δ): 1.11(6H,m), 1.90(3H,s), 3.35(1H,m), 3.71(1H,br), 4.43(4H,d), 4.64 (1H,br), 7.35(5H,m)

### EXAMPLE 35

### Preparation of (S )-3-N-cyclopropyl carbamoyl 2-phenyl propanol acetate

Except for using cyclopropyl amine instead of aqueous ammonia, the same procedure with that of Example 31 was repeated.

A concentrated reaction mixture thus obtained was separated by column chromatography using a mixture of ethyl acetate/n-hexane (1:1) as a mobile phase, to obtain 64.2 g of (S)-3-N-cyclopropyl carbamoyl-2-phenyl propanol acetate as a colorless liquid.
Yield 90%
H-NMR(CDCl₃, 200MHz), ppm (δ): 0.45-0.81(6H,m), 2.01(3H,s), 2.52(1H,br), 3.37(1H,q), 4.45(4H,d), 1.87(1H,br), 7.32(5H,m)

### EXAMPLE 36

### Preparation of (S)-3-N-morphoryl carbamoyl-2-phenyl propanol acetate

Except for using morphorine instead of aqueous ammonia, the same procedure with that of Example 31 was repeated.

A concentrated reaction mixture thus obtained was separated by column chromatography using a mixture of ethyl acetate/n-hexane (1:1) as a mobile phase, to obtain 75.2 g of (S)-3-N-morphorylcarbamoyl-2-phenyl propanol acetate as a colorless liquid.
Yield 95%
H-NMR(CDCl₃, 200MHz), ppm(δ): 2.00(3H,s), 3.25-3.73(9H,br), 4.31(4H,d), 7.27(5H,m)

### EXAMPLE 37

### Preparation of (S)-3-N-methylcarbamoyl-2-(m-chloro phenyl) propanol acetate

A well dried 1,000 ml flask equipped with a thermometer was purged with nitrogen gas, to completely take off moisture and air from the inside of the flask. This purging was continued for 30 min, after which 500 ml of purified methylene chloride was poured into the flask. 58.9 g of (R)-3-acetoxy-2-(m-chlorophenyl) propanol was well dissolved in the solvent and made to be homogeneous by stirring for 5 min. While being maintained at 0°C, the homogeneous solution was slowly added with 49.8 g of carbonyl diimidazole. After the starting material completely disappeared as measured by thin layer chromatography, 60 ml of an aqueous methylamine solution was slowly added to the reaction solution in order to progress the reaction

To determine when the reaction would be terminated, the reaction was under double monitoring of thin layer chromatography and liquid chromatography. Roughly, it took about 2 hours to finish the reaction.

After completion of reaction, 500 ml of distilled water was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, to obtain 71.2 g of (S)-3-N-methylcarbamoyl)-2-(m-chlorophenyl) propanol acetate.
Yield 90%
H-NMR(DMSO, 200MHz), ppm(δ): 2.12(3H,s), 2.90(d,3H), 3.31(1H,q), 4.12-4.31 (4H,m), 5.52(1H,br), 7.28-7.41(4H,m)

### EXAMPLE 38

### Preparation of (S)-3-Carbamoyl-2-phenyl propanol

A well dried 1,000 ml flask equipped with a thermometer was purged with nitrogen gas, to completely take off moisture and air from the inside of the flask. This purging was continued for 30 min, after which 500 ml of methanol was poured into the flask. 47.4 g of (S)-3-carbamoyl-2-phenyl propanol acetate prepared in Example 31 was well dissolved in the solvent and made to be homogeneous by stirring for 30 min. While being maintained at 0°C, the homogeneous solution was slowly added with 3.9 g of potassium cyanide. The resulting reaction mixture was heated to room temperature with stirring. To determine when the reaction would be terminated, the reaction was under double monitoring of thin layer chromatography and liquid chromatography. Roughly, it took about 2 hours to finish the reaction.

After completion of the reaction, 200 ml of saturated ammonium chloride solution was added. Then, using a rotary evaporator, unreacted methanol was completely removed from the reaction solution. 200 ml of distilled water and 400 ml of ethyl acetate were added for solvent extraction. The organic layer thus obtained was dried over anhydrous magnesium sulfate and distilled off the solvent by a rotary evaporator, to give a yellow liquid.

This liquid obtained was added with 200 ml of toluene and stored in a refrigerator for 24 hrs, to produce 35.5 g of (S)-3-carbamoyl-2-phenyl propanol as a while solid.
Yield 91%
H-NMR(CDCl₃, 200MHz), ppm(δ): 2.58(1H,t), 3.11(1H,q), 3.82(2H,d), 4.39(2H,d), 4.91(2H,br), 7.27(5H,m)

### EXAMPLE 39

### Preparation of (S)-3-N-methylcarbamoyl-2-phenyl propanol

Except for using (S)-3-N-methylcarbamoyl-2-phenyl propanol acetate, instead of (S)-3-carbamoyl-2-phenyl propanol acetate, as the starting material, the same procedure with that of Example 38 was repeated.

A concentrated reaction mixture thus obtained was separated by column chromatography using a mixture of ethyl acetate/n-hexane (1:1) as a mobile phase, to obtain 37.2 g of (S)-3-N-methylcarbamoyl-2-phenyl propanol as a white solid.
Yield 89%
H-NMR(DMSO, 200MHz), ppm(δ): 2.52(3H,d), 3.01(1H,q), 3.61(2H,t), 4.18(2H,m), 4.75(1H,t), 6.98(1H,d), 7.28(5H,m)

### EXAMPLE 40

### Preparation of (S)-3-N,N'-Dimethylcarbamoyl -2-phenyl propanol

Except for using (S)-3-N,N-dimethylcarbamoyl-2-phenyl propanol acetate, instead of (S)-3-carbamoyl 2-phenyl propanol acetate, as the starting material, the same procedure with that of Example 38 was repeated.

A concentrated reaction mixture thus obtained was separated by column chromatography using a mixture of ethyl acetate/n-hexane (1:1) as a mobile phase, to obtain 38.8 g of (S)-3-N.N'-dimethylcarbamoyl-2-phenyl propanol as a colorless liquid.
Yield 87%
H-NMR(DMSO, 200MHz), ppm(δ): 2.61(6H,d), 3.10(1H,q), 3.73(2H,d), 4.12(2H,m), 4.74(1H,t), 7.34(5H,m)

### EXAMPLE 41

### Preparation of (S)-3-N-Isopropyl carbamoyl-2-phenyl propanol

Except for using (S)-3-N-isopropyl carbamoyl-2-phenyl propanol acetate, instead of (S)-3-carbamoyl-2-phenyl propanol acetate, as the starting material, the same procedure with that of Example 38 was repeated.

A concentrated reaction mixture thus obtained was separated by column chromatography using a mixture of ethyl acetate/n-hexane (1:1) as a mobile phase, to obtain 41.7 g of (S)-3-N-isopropyl carbamoyl-2-phenyl propanol as a white solid.
Yield 88%
H-NMR(DMSO, 200MHz), ppm(δ): 1.00(6H,d), 2.52(1H,d), 2.95(1H,t), 3.62(2H,d), 4.18(2H,m), 4.74(1H,t), 6.91(1H,d), 7.28(5H,m)

### EXAMPLE 42

### Preparation of (S)-3-N-Cyclopropyl carbamoyl2-phenyl propanol

Except for using (S)-3-N-cyclopropyl carbamoyl-2-phenyl propanol acetate, instead of (S)-3-carbamoyl 2-phenyl propanol acetate, as the starting material, the same procedure with that of Example 38 was repeated.

A concentrated reaction mixture thus obtained was separated by column chromatography using a mixture of ethyl acetate/n-hexane (1:1) as a mobile phase, to obtain 42.8 g of (S)-3-N-cyclopropyl carbamoyl-2-phenyl propanol as a white solid.
Yield 91%
H-NMR(DMSO, 200MHz), ppm(δ): 0.35-0.55(4H,m), 2.52(1H,s), 3.01(1H,t), 3.61(2H,d), 4.25(2H,m), 4.76(1H,t), 7.14(1H,d), 7.28(5H,m)

### EXAMPLE 43

### Preparation of (S)-3-N'-Morphoryl carbamoyl-2-phenyl propanol

Except for using (S)-3-N-morphoryl carbamoyl-2-phenyl propanol acetate, instead of (S)-3-carbamoyl-2-phenyl propanol acetate, as the starting material, the same procedure with that of Example 38 was repeated.

A concentrated reaction mixture thus obtained was separated by column chromatography using a mixture of ethyl acetate/n-hexane (1:1) as a mobile phase, to obtain 47.2 g of (S)-3-N-morphorylcarbamoyl-2-phenyl propanol as a white solid.
Yield 89%
H-NMR(DMSO, 200MHz), ppm(δ): 2.82(1H,br), 3.15(1H,q), 3.27-3.60(8H,br), 3.79(2H,d), 4.35(2H,d), 7.25(5H,m)

### EXAMPLE 44

### Preparation of (S)-3-N-Methylcarbamoyl-2-(m-chloro phenyl) propanol

A well dried 1,000 ml flask equipped with a thermometer was purged with nitrogen gas, to completely take off moisture and air from the inside of the flask. This purging was continued for 30 min, after which 500 ml of methanol was poured into the flask. 69.5 g of (S)-3-N-methylcarbamoyl-2-(m-chlorophenyl) propanol acetate prepared in Example 37 was well dissolved in the solvent and made to be homogeneous by stirring for 30 min. While being maintained at 0°C, the homogeneous solution was slowly added with 3.9 g of potassium cyanide. The resulting reaction mixture was heated to room temperature with stirring. To determine when the reaction would be terminated, the reaction was under double monitoring of thin layer chromatography and liquid chromatography. Roughly, it took about 2 hours to finish the reaction.

After completion of the reaction, 200 ml of saturated ammonium chloride solution was added. Then, using a rotary evaporator, unreacted methanol was completely removed from the reaction solution. 200 ml of distilled water and 400 ml of ethyl acetate were added for solvent extraction. The organic layer thus obtained was dried over anhydrous magnesium sulfate and distilled off the solvent by a rotary evaporator, to give a yellow liquid.

This liquid obtained was added with 300 ml of toluene and stored in a refrigerator for 24 hrs, to produce 50.5 g of (S)-3-N-methylcarbamoyl-2-(m-chlorophenyl) propanol as a white solid.
Yield 85%
H-NMR(CDCl₃, 200MHz), ppm(δ): 2.41(3H,d), 2.51(1H,t), 3.20(1H,q), 3.92-4.15(4H,br), 5.58(1H,br), 7.30-7.49(4H,m)

### EXAMPLE 45

### Preparation of (R)-3-Carbamoyl-2-phenyl propanol sulfamate

A well dried 2,000 ml flask equipped with a thermometer was purged with nitrogen gas, to completely take off moisture and air from the inside of the flask. This purging was continued for 30 min, after which 1,200 ml of purified acetonitrile was poured into the flask. 58.5 g of (S)-3-carbamoyl-2-phenyl propanol prepared in Example 38 and 156.8 ml of diisopropyl ethylamine were well dissolved in the solvent and made to be homogeneous by stirring for 30 min. While being maintained at 0°C, the homogeneous solution was slowly added with 69.3 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, unreacted acetonitrile was completely removed from the reaction solution using a rotary evaporator. Then, 600 ml of distilled water and 600 ml of ethyl acetate were added for solvent extraction. The organic layer thus obtained was dried over anhydrous magnesium sulfate and distilled off the solvent by a rotary evaporator, to give a yellow liquid.

This liquid obtained was separated by column chromatography using a mixture of ethyl acetate/n-hexane (1:1) as a mobile phase, to obtain 73.2 g of (R)-3-carbamoyl-2-phenyl propanol sulfamates a white solid.
Yield 89%
H-NMR(DMSO, 200MHz), ppm(δ): 3.35(1H,q), 4.16(2H,d), 4.34(2H,d), 6.57(2H,br), 7.39(5H,s), 7.58(2H,s)

### EXAMPLE 46

### Preparation of (R)-3-N-Methylcarbamoyl-2-phenyl propanol sulfamate

Except for using (S)-3-N-methylcarbamoyl-2-phenyl propanol, instead of (S)-3-carbamoyl-2-phenyl)propanol, as the starting material, the same procedure with that of Example 45 was repeated.

A concentrated reaction mixture thus obtained was separated by column chromatography using a mixture of ethyl acetate/n-hexane (1:1) as a mobile phase, to obtain 76.9 g of (R)-3-N-methylcarbamoyl-2-phenyl propanol sulfamate as a white solid.
Yield 89%
H-NMR(DMSO, 200MHz), ppm(δ): 2.54(3H,d), 3.35(1H,q), 4.29(4H,m), 6.98(1H,br), 7.29(5H,s), 7.53(2H,s)

### EXAMPLE 47

### Preparation of (R)-3-N,N'-Dimethylcarbamoyl-2-phenyl propanol sulfamate

Except for using (S)-3-N,N'-dimethylcarbamoyl-2-phenyl propanol, instead of (S)-3-carbamoyl-2-phenyl propanol, as the starting material, the same procedure with that of Example 45 was repeated.

A concentrated reaction mixture thus obtained was separated by column chromatography using a mixture of ethyl acetate/n-hexane (1:1) as a mobile phase, to obtain 77.9 g of (R)-3-N,N'-dimethylcarbamoyl-2-phenyl propanol sulfamate as a white solid.
Yield 86%
H-NMR(CDCl₃, 200MHz), ppm(δ): 2.84(6H,d), 3.38(1H,q), 4.39(4H,m), 4.93(2H,s), 7.21(5H,d)

### EXAMPLE 48

### Preparation of (R)-3-N-Isopropyl carbamoyl-2-phenyl propanol sulfamate

Except for using (S )-3-N-isopropyl carbamoyl-2-phenyl propanol, instead of (S)-3-carbamoyl-2-phenyl propanol, as the starting material, the same procedure with that of Example 45 was repeated.

A concentrated reaction mixture thus obtained was separated by column chromatopraphy using a mixture of ethyl acetate/n-hexane (1:1) as a mobile phase, to obtain 79.6 g of (R)-3-N-isopropyl carbamoyl-2-phenyl propanol sulfamate as a white solid.
Yield 84%
H-NMR(DMSO, 200MHz), ppm (δ): 1.01(6H,m), 3.32(1H,m), 3.55(1H,q), 4.18(2H,d), 4.29(2H,d), 6.95(1H,d), 7.34(5H,s), 7.56(2H,s)

### EXAMPLE 49

### Preparation of (R)-3-N-Cyclopropyl carbamoyl-2-phenyl propanol sulfamate

Except for using (S)-3-N-cyclopropyl carbamoyl-2-phenyl propanol, instead of (S)-3-carbamoyl-2-phenyl propanol, as the starting material, the same procedure with that of Example 45 was repeated.

A concentrated reaction mixture thus obtained was separated by column chromatography using a mixture of ethyl acetate/n-hexane (1:1) as a mobile phase, to produce 84.8 g of (R)-3-N-cyclopropyl carbamoyl-2-phenyl propanol sulfamate as a white solid.
Yield 90%
H-NMR(DMSO, 200MHz) ppm(δ): 0.35-0.58(4H,m), 2.51(1H,s), 3.35(1H,q), 4.16-4.36(4H,m), 7.15(1H,br), 7.29(5H,s), 7.52(2H,s)

### EXAMPLE 50

### Preparation of (R)-3-N-Morphoryl carbamoyl-2-phenyl propanol sulfamate

Except for using (S)-3-N-morphoryl carbamoyl-2-phenyl propanol, instead of (S)-3-carbamoyl-2-phenyl propanol, as the starting material, the same procedure with that of Example 45 was repeated.

The liquid thus obtained was separated by column chromatography using a mixture of ethyl acetate/n-hexane (1:1) as a mobile phase, to produce 87.8 g of (R)-3-N-morphorylcarbamoyl-2-phenyl propanol sulfamate as a white solid.
Yield 85%
H-NMR(DMSO, 200MHz) ppm(δ): 3.05(1H,q), 3.20-3.50(8H,br), 4.15(4H,m), 4.43(2H,s), 7.13(5H,m)

### EXAMPLE 51

### Preparation of (R)-3-N-Methylcarbamoyl-2-(m-chloro phenyl) propanol sulfamate

A well dried 2,000 ml flask equipped with a thermometer was purged with nitrogen gas, to completely take off moisture and air from the inside of the flask. This purging was continued for 30 min, after which 1,200 ml of purified acetonitrile was poured into the flask. 73.1 g of (S)-3-N-methylcarbamoyl-2-(m-chlorophenyl) propanol prepared in Example 44 and 156.8 ml of diisopropyl ethylamine were well dissolved in the solvent and made to be homogeneous by stirring for 30 min. While being maintained at 0°C, the homogeneous solution was slowly added with 69.3 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, acetonitrile remaining was completely removed from the reaction mixture by using a rotary evaporator. 600 ml of distilled water and 600 ml of ethyl acetate were added for solvent extraction. The organic layer thus obtained was dried over anhydrous magnesium sulfate and distilled off the solvent by a rotary evaporator, to give a yellow liquid.

This liquid obtained was separated by column chromatography using a mixture of ethyl acetate/n-hexane (1:1) as a mobile phase, to produce 73.2 g of (R)-3-N-methylcarbamoyl-2-(m-chlorophenyl) propanol sulfamate as a white solid.
Yield 89%
H-NMR(DMSO, 200 MHz), ppm (δ): 2.50(3H,d), 3.35(1H,q), 4.28(4H,m), 6.52(1H,br), 7.38(4H,m), 7.56(2H,s)

### EXAMPLE 52

### Preparation of (S)-3-Acetoxy-2-phenyl-1,3-propandiol sulfamate

A well dried 1,000 ml flask equipped with a thermometer was purged with nitrogen gas, to completely take off moisture and air from the inside of the flask. This purging was continued for 30 min, after which 300 ml of purified acetonitrile was poured into the flask. 50 g of (R)-3-acetoxy-2-phenyl propanol and 61 ml of pyridine were well dissolved in the solvent and made to be homogeneous by stirring for 30 min. While being maintained at 0°C, the homogeneous solution was slowly added with 59.6 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, acetonitrile was completely removed from the reaction solution using a rotary evaporator. Then, 300 ml of distilled water and 300 ml of ethyl acetate were added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, to produce 61.3 g of (S)-3-acetoxy-2-phenyl-1,3-propandiol sulfamate as a white solid.
Yield 88%
H-NMR(DMSO, 200MHz), ppm(δ): 1.95(3H,s), 3.36(1H,q), 4.28(4H,d), 7.28(5H,s), 7.53(2H,s)

### EXAMPLE 53

### Preparation of (S)-2-Phenyl-1,3-propandiol monosulfamate

A well dried 1,000 ml flask equipped with a thermometer was purged with nitrogen gas, to completely take off moisture and air from the inside of the flask. This purging was continued for 30 min, after which 500 ml of methanol was poured into the flask. 27.3 g of (S)-3-acetoxy-2-phenyl-1,3-propandiol sulfamate prepared in Example 52 was well dissolved in the solvent and made to be homogeneous by stirring for 30 min. While being maintained at 0°C, the homogeneous solution was slowly added with 3.3 g of potassium cyanide. The resulting reaction mixture was heated to room temperature with stirring. To determine when the reaction would be terminated, the reaction was under double monitoring of thin layer chromatography and liquid chromatography. Roughly, it took about 4 hours to finish the reaction.

After completion of the reaction, 50 ml of saturated ammonium chloride solution was added. Then, using a rotary evaporator, unreacted methanol was completely removed from the reaction solution. 200 ml of distilled water and 200 ml of ethyl acetate were added for solvent extraction. The organic layer thus obtained was dried over anhydrous magnesium sulfate and distilled off the solvent by a rotary evaporator, to give a yellow solid.

This solid was washed with 50 ml of methylene chloride and filtered to produce 19 g of (S)-2-phenyl-1,3-propandiol monosulfamate as a white solid.
Yield 81%
H-NMR(DMSO, 200MHz), ppm(δ): 3.12(1H,q), 3.61(2H,d), 4.24(2H,m), 4.86(1H,t), 7.15(5H,m), 7.46 (2H,s)

### EXAMPLE 54

### Preparation of (S)-3-N-Methylcarbamoyl-2-phenyl propanol sulfamate

A well dried 1,000 ml flask equipped with a thermometer was purged with nitrogen gas, to completely take off moisture and air from the inside of the flask. This purging was continued for 30 min, after which 300 ml of purified methylene chloride was poured into the flask. 23.3 g of (S)-2-phenyl-1,3-propandiol monosulfamate prepared in Example 53 was well dissolved in the solvent and made to be homogeneous by stirring for 30 min. While being maintained at 0°C, the homogeneous solution was slowly added with 19.4 g of carbonyl diimidazole. The resulting reaction mixture was slowly heated to room temperature with stirring. To determine when the reaction would be terminated, the reaction was under double monitoring of thin layer chromatography and liquid chromatography. Roughly, it took about 15 mins to finish the reaction. After the starting material completely disappeared, 47 ml of methyl amine aqueous solution was slowly introduced through a syringe with stirring at room temperature. It took about 2 hours to completely terminate this reaction.

Then, using a rotary evaporator, the solvent remaining was completely removed from the reaction solution. 200 ml of distilled water and 200 ml of ethyl acetate were added for solvent extraction. The organic layer thus obtained was dried over anhydrous magnesium sulfate and distilled off the solvent by a rotary evaporator, to give a yellow liquid.

This liquid was added with 150 ml of ethyl ether and stored in a refrigerator for 24 hrs, to produce 24.4 g of (S)-3-N-methylcarbamoyl-2-phenyl propanol sulfamate.
Yield 85%
H-NMR(DMSO, 200MHz), ppm(δ): 2.54(3H,d), 3.35(1H,q), 4.29(4H,m), 6.98(1H,br), 7.29(5H,s), 7.53(2H,s)

### EXAMPLE 55

### Preparation of (S)-3-Carbamoyl-2-phenyl propanol sulfamate

Except for using aqueous ammonia instead of methylamine, the same procedure with that of Example 54 was repeated.

A liquid obtained was added with 150 ml of ethyl ether and stored in a refrigerator to produce 27.0 g of (S)-3-carbamoyl-2-phenyl propanol sulfamate as a white solid.
Yield 80%
H-NMR(DMSO, 200MHz), ppm(δ): 3.35(1H,q), 4.16(2H,d), 4.34(2H,d), 6.57(2H,br), 7.39(5H,s), 7.58(2H,s), 1.98(3H,s), 3.28(1H,q)

### EXAMPLE 56

### Preparation of (S)-3-N,N'-Dimethylcarbamoyl-2-phenyl propanol sulfamate

Except for using dimethylamine instead of methylamine, the same procedure with that of Example 54 was repeated.

A liquid obtained was added with 150 ml of ethylether and stored in a refrigerator for 24 hrs, to produce 27.0 g of (S)-3-N,N'-dimethylcarbamoyl-2-phenyl propanol sulfamate as a white solid.
Yield 80%
H-NMR(DMSO, 200MHz), ppm (δ): 2.84(6H,d), 3.38(1H,q), 4.39(4H,m), 4.93(2H,s), 7.21(5H,d)

### EXAMPLE 57

### Preparation of (S)-3-N-Isopropyl carbamoyl 2-phenyl propanol sulfamate

Except for using isopropyl amine instead of methyl amine the same procedure with that of Example 54 was repeated.

A liquid obtained was added with 150 ml of ethylether and stored in a refrigerator for 24 hrs, to produce 26.0 g of (S)-3-N-isopropyl carbamoyl-2-phenyl propanol sulfamate.
Yield 82%
H-NMR(DMSO, 200MHz), ppm(δ): 1.01(6H,m), 3.32(1H,m), 3.55(1H,q), 4.18(2H,d), 4.29(2H,d), 6.95(1H,d), 7.34(5H,s)

### EXAMPLE 58

### Preparation of (S)-3-N-Cyclopropyl carbamoyl-2-phenyl propanol sulfamate

Except for using cyclopropyl amine instead of methyl amine, the same procedure with that of Example 54 was repeated.

A liquid obtained was added with 150 ml of ethylether and stored in a refrigerator for 24 hrs, to produce 27.6 g of (S)-3-N-cyclopropyl carbamoyl-2-phenyl propanol sulfamate.
Yield 88%
H-NMR(DMSO, 200MHz) ppm(δ): 0.35-0.58(4H,m), 2.51(1H,s), 3.35(1H,q), 4.16-4.36(4H,m), 7.15(1H,br), 7.29(5H,s)

### EXAMPLE 59

### Preparation of (S)-3-N-Morphoryl carbamoyl-2-phenyl propanol sulfamate

Except for using morphorine instead of methyl amine, the same procedure with that of Example 54 was repeated

A liquid thus obtained was added with 500 ml of ethylether and stored in a refrigerator for 24 hrs, to produce 33 g of (S)-3-N-morphoryl carbamoyl-2-phenyl propanol sulfamate.
Yield 96%
H-NMR(DMSO, 200MHz) ppm(δ): 3.05(1H,q) 3.20-3.50(8H,br), 4.15(4H,m), 4.43(2H,s), 7.13(5H,m)

### EXAMPLE 60

### Preparation of (R)-3-Carbamoyl-2-phenyl propanol acetate

A well dried 1,000 ml flask equipped with a thermometer was purged with nitrogen gas, to completely take off moisture and air from the inside of the flask. This purging was continued for 30 min, after which 500 ml of purified methylene chloride was poured into the flask. 50 g of (S)-3-acetoxy 2-phenyl propanol was well dissolved in the solvent and made to be homogeneous by stirring for 5 min. While being maintained at 0°C, the homogeneous solution was slowly added with 49.8 g of carbonyl diimidazole. After the starting material completely disappeared as measured by thin layer chromatography, 43.8 ml of aqueous ammonia was slowly added to the reaction solution in order to progress the reaction.

To determine when the reaction would be terminated, the reaction was under double monitering of thin layer chromatography and liquid chromatography. Roughly, it took about 2 hours to finish the reaction.

After completion of reaction, 500 ml of distilled water was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, to obtain 55 g of (R)-3-carbamoyl-2-phenyl propanol acetate.
Yield 90%
H-NMR(DMSO, 200MHz), ppm(δ): 1.98(3H,s), 3.28(1H,q), 4.28(4H,d), 5.21(2H,br), 7.27(5H,m)

### EXAMPLE 61

### Preparation of (R)-3-N-methylcarbamoyl-2-phenyl propanol acetate

Except for rising methylamine instead of aqueous ammonia, the same procedure with that of Example 60 was repeated.

A concentrated reaction mixture thus obtained was separated by column chromatography using a mixture of ethyl acetate/n-hexane (1:1) as a mobile phase, to obtain 62.1 g of (R)-3-N-methylcarbamoyl-2-phenyl propanol acetate as a colorless liquid.
Yield 96%
H-NMR(CDCl₃, 200MHz), ppm(δ): 1.95(3H,s), 2.75(3H,d), 3.28(1H,q), 4.21(4H,d), 5.15(1H,br), 7.27(5H,m)

### EXAMPLE 62

### Preparation of (R)-3-N,N'-dimethylcarbamoyl-2-phenyl propanol acetate

Except for using dimethylamine instead of aqueous ammonia, the same procedure with that of Example 60 was repeated.

A concentrated reaction mixture thus obtained was separated by column chromatography using a mixture of ethyl acetate/n-hexane (1:1) as a mobile phase, to obtain 61.4 g of (R)-3-N,N'-dimethylcarbamoyl-2-phenyl propanol acetate as a colorless liquid.
Yield 90%
H-NMR(CDCl₃, 200MHz), ppm(δ): 1.97(3H,s), 2.78(6H,d), 3.34(1H,q), 4.17(2H,d), 4.39(2H,d), 7.32(5H,m)

### EXAMPLE 63

### Preparation of (R)-3-N-isopropyl carbamoyl-2-phenyl propanol acetate

Except for using isopropylamine instead of aqueous ammonia, the same procedure with that of Example 60 was repeated.

A concentrated reaction mixture thus obtained was separated by column chromatography using a mixture of ethyl acetate/n-hexane (1:1) as a mobile phase, to obtain 65.5 g of (R)-3-N-isopropylcarbamoyl-2-phenyl propanol acetate as a colorless liquid.
Yield 91%
H-NMR(CDCl₃, 200MHz), ppm(δ): 1.11(6H,m), 1.90(3H,s), 3.35(1H,m), 3.71(1H,br), 4.43(4H,d), 4.64(1H,br), 7.35(5H,m)

### EXAMPLE 64

### Preparation of (R )-3-N-cyclopropyl carbamoyl-2-phenyl propanol acetate

Except for using cyclopropyl amine instead of aqueous ammonia, the same procedure with that of Example 60 was repeated.

A concentrated reaction mixture thus obtained was separated by column chromatography using a mixture of ethyl acetate/n-hexane (1:1) as a mobile phase, to obtain 66.3 g of (H)-3-N-cyclopropyl carbamoyl-2-phenyl propanol acetate as a colorless liquid.
Yield 93%
M-NMR(CDCl₃, 200MHz), ppm(δ): 0.45-0.81(6H,m), 2.01(3H,s), 2.52(1H,br), 3.37(1H,q), 4.45(4H,d), 4.87(1H,br), 7.32(5H,m)

### EXAMPLE 65

### Preparation of (R)-3-N-morphoryl carbamoyl 2-phenyl propanol acetate

Except for using morphorine instead of aqueous ammonia, the same procedure with that of Example 60 was repeated.

A concentrated reaction mixture thus obtained was separated by column chromatography using a mixture of ethyl acetate/n-hexane (1:1) as a mobile phase, to obtain 70.5 g of (R)-3-N-morphorylcarbamoyl-2-phenyl propanol acetate as a colorless liquid.
Yield 89%
H-NMR(CDCl₃, 200MHz), ppm(δ): 2.00(3H,s), 3.25-3.73(9H,br), 4.31(4H,d), 7.27(5H,m)

### EXAMPLE 66

### Preparation of (R)-3-N-methylcarbamoyl-2-(p-methoxyphenyl) propanol acetate

A well dried 1,000 ml flask equipped with a thermometer was purged with nitrogen gas, to completely take off moisture and air from the inside of the flask. This purging was continued for 30 min, after which 500 ml of purified methylene chloride was poured into the flask. 57.8 g of (S)-3-acetoxy-2-(p-methoxyphenyl) propanol was well dissolved in the solvent and made to be homogeneous by stirring for 5 min. While being maintained at 0°C, the homogeneous solution was slowly added with 49.8 g of carbonyl diimidazole. After the starting material completely disappeared as measured by thin layer chromatography, 60 ml of an aqueous methylamine solution was slowly added to the reaction solution in order to progress the reaction.

To determine when the reaction would be terminated, the reaction was under double monitering of thin layer chromatography and liquid chromatography. Roughly, it took about 2 hours to finish the reaction.

After completion of reaction, 500 ml of distilled water was added for solvent extraction. The organic layer thus obtained was dried over anhydrous magnesium sulfate and distilled off the solvent by a rotary evaporator, to give a yellow liquid.

This concentrated liquid mixture was separated by column chromatography using a mixture of ethyl acetate/n-hexane (1:1) as a mobile phase, to obtain 69.6 g of (R)-3-N-methylcarbamoyl-2-(p-methoxyphenyl) propanol acetate.
Yield 96%
H-NMR(DMSO, 200MHz), ppm(δ): 2.12(3H,s), 2.85(d,3H), 3.25(1H,q), 4.20-4.32(4H,m), 5.24 (1H,br), 6.89(2H,d), 7.23(2H,d)

### EXAMPLE 67

### Preparation of (R)-3-Carbamoyl-2-phenyl propanol

A well dried 1,000 ml flask equipped with a thermometer was purged with nitrogen gas, to completely take off moisture and air from the inside of the flask. This purging was continued for 30 min, after which 500 ml of methanol was poured into the flask. 47.4 g of (R)-3-carbamoyl-2-phenyl propanol acetate prepared in Example 60 was well dissolved in the solvent and made to be homogeneous by stirring for 30 min. While being maintained at 0°C, the homogeneous solution was slowly added with 3.9 g of potassium cyanide. The resulting reaction mixture was heated to room temperature with stirring. To determine when the reaction would be terminated, the reaction was under double monitering of thin layer chromatography and liquid chromatography. Roughly, it took about 2 hours to finish the reaction.

After completion of the reaction, 200 ml of saturated ammonium chloride solution was added. Then, using a rotary evaporator, unreacted methanol was completely removed from the reaction solution. 200 ml of distilled water and 400 ml of ethyl acetate were added for solvent extraction. The organic layer thus obtained was dried over anhydrous magnesium sulfate and distilled off the solvent by a rotary evaporator, to give a yellow liquid.

This liquid obtained was added with 200 ml of toluene and stored in a refrigerator for 24 hrs, to produce 36.7 g of (R)-3-carbamoyl-2-phenyl propanol as a white solid.
Yield 94%
H-NMR(CDCl₃, 200MHz), ppm(δ): 2.58(1H,t), 3.11(1H,q), 3.82(2H,d), 4.39(2H,d), 4.91(2H,br), 7.27(5H,m)

### EXAMPLE 68

### Preparation of (R)-3-N-methylcarbamoyl-2-phenyl propanol

Except for using (R)-3-N-methylcarbamoyl-2-phenyl propanol acetate, instead of (R)3-carbamoyl-2-phenyl propanol acetate, as the starting material, the same procedure with that of Example 67 was repeated.

A concentrated reaction mixture thus obtained was separated by column chromatography using a mixture of ethyl acetate/n-hexane (1:1) as a mobile phase, to obtain 38.9 g of (R)-3-N-methylcarbamoyl-2-phenyl propanol as a while solid.
Yield 93%
H-NMR(DMSO, 200MHz), ppm (δ): 2.52(3H,d), 3.01(1H,q), 3.61(2H,t), 4.18(2H,m), 4.75(1H,t), 6.98(1H,d), 7.28(5H,m)

### EXAMPLE 69

### Preparation of (R)-3-N,N'-Dimethylcarbamoyl-2-phenyl propanol

Except for using (R)-3-N,N'-dimethylcarbamoyl-2-phenyl propanol acetate, instead of (R)-3-carbamoyl-2-phenyl propanol acetate, as the starting material, the same procedure with that of Example 67 was repeated.

A concentrated reaction mixture thus obtained was separated by column chromatography using a mixture of ethyl acetate/n-hexane (1:1) as a mobile phase, to obtain 41.5 g of (R)-3-N,N'-dimethylcarbamoyl-2-phenyl propanol as a colorless liquid.
Yield 93%
H-NMR(DMSO, 200MHz), ppm(δ): 2.61(6H,d), 3.10(1H,q), 3.73(2H,d), 4.12(2H,m), 4.74(1H,t), 7.34(5H,m)

### EXAMPLE 70

### Preparation of (R)-3-N-Isopropyl carbamoyl-2-phenyl propanol

Except for using (R)-3-N-isopropyl carbamoyl-2-phenyl propanol acetate, instead of (R)-3-carbamoyl-2-phenyl propanol acetate, as the starting material, the same procedure with that of Example 67 was repeated.

A concentrated reaction mixture thus obtained was separated by column chromatography using a mixture of ethyl acetate/n-hexane (1:1) as a mobile phase, to obtain 42.6 g of (R)-3-N-isopropyl carbamoyl-2-phenyl propanol as a while solid.
Yield 90%
H-NMR(DMSO, 200MHz), ppm(δ): 1.00(6H,d), 2.52(1H,d), 2.95(1H,t), 3.62(2H,d), 4.18(2H,m), 4.74(1H,t), 6.91(1H,d) 7.28(5H,m)

### EXAMPLE 71

### Preparation of (R)-3-N-Cyclopropyl carbamoyl-2-phenyl propanol

Except for using (R)-3-N-cyclopropyl carbamoyl 2 phenyl propanol acetate, instead of (R)-3-carbamoyl 2-phenyl propanol acetate, as the starting material, the same procedure with that of Example 67 was repeated.

A concentrated reaction mixture thus obtained was separated by column chromatography using a mixture of ethyl acetate/n-hexane (1:1) as a mobile phase, to obtain 44.2 g of (R)-3-N-cyclopropyl carbamoyl-2-phenyl propanol as a white solid.
Yield 94%
H-NMR(DMSO, 200MHz), ppm(δ): 0.35-0.55(4H,m), 2.52(1H,s), 3.01(1H,t), 3.61(2H,d), 4.25(2H,m), 4.76(1H,t), 7.14(1H,d), 7.28(5H,m)

### EXAMPLE 72

### Preparation of (R)-3-N-Morphoryl carbamoyl-2-phenyl propanol

Except for using (R)-3-N-morphoryl carbamoyl-2-phenyl propanol acetate, instead of (R)-3-carbamoyl-2-phenyl propanol acetate, as the starting material, the same procedure with that of Example 67 was repeated.

A concentrated reaction mixture thus obtained was separated by column chromatography using a mixture of ethyl acetate/n-hexane (1:1) as a mobile phase, to obtain 48.3 g of (R)-3-N-morphorylcarbamoyl-2-phenyl propanol as a white solid.
Yield 91%
H-NMR(DMSO, 200MHz), ppm(δ): 2.82(1H,br), 3.15(1H,q), 3.27-3.60(8H,br), 3.79(2H,d), 4.35(2H,d), 7.25(5H,m)

### EXAMPLE 73

### Preparation of (R)-3-N-Methylcarbamoyl-2-(p-methoxyphenyl) propanol

A well dried 1,000 ml flask equipped with a thermometer was purged with nitrogen gas, to completely take off moisture and air from the inside of the flask. This purging was continued for 30 min, after which 500 ml of methanol was poured into the flask. 56.3 g of (R)-3-N-methylcarbamoyl-2-(p-methoxyphenyl) propanol acetate prepared in Example 66 was well dissolved in the solvent and made to be homogeneous by stirring for 30 min. While being maintained at 0°C, the homogeneous solution was slowly added with 3.9 g of potassium cyanide. The resulting reaction mixture was heated to room temperature with stirring. To determine when the reaction would he terminated, the reaction was under double monitoring of thin layer chromatography and liquid chromatography. Roughly, it took about 2 hours to finish the reaction.

After completion of the reaction, 200 ml of saturated ammonium chloride solution was added. Then, using a rotary evaporator, unreacted methanol was completely removed from the reaction solution. 200 ml of distilled water and 400 ml of ethyl acetate were added for solvent extraction. The organic layer thus obtained was dried over anhydrous magnesium sulfate and distilled off the solvent by a rotary evaporator, to give a yellow liquid.

This liquid obtained was added with 100 ml of toluene and stored in a refrigerator for 24 hrs, to produce 40.7 g of (R)-3-N-methylcarbamoyl-2-(p-melhoxyphenyl) propanol as a while solid.
Yield 85%
H-NMR(CDCl₃, 200MHz, ppm(δ): 2.39(3H,d), 2.51(1H,t), 3.24(1H,q), 3.75-4.05(4H,br), 5.43(1H,br), 6.91(2H,d), 7.20(2H,d)

### EXAMPLE 74

### Preparation of (S)-3-Carbamoyl-2-phenyl propanol sulfamate

A well dried 2,000 ml flask equipped with a thermometer was purged with nitrogen gas, to completely take off moisture and air from the inside of the flask. This purging was continued for 30 min, after which 1,200 ml of purified acetonitrile was poured into the flask. 58.5 g of (R)-3-carbamoyl-2-phenyl propanol prepared in Example 67 and 156.8 ml of diisopropyl ethylamine were well dissolved in the solvent and made to be homogeneous by stirring for 30 min. While being maintained at 0°C, the homogeneous solution was slowly added with 69.3 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, unreacted acetonitrile was completely removed from the reaction solution using a rotary evaporator. Then, 600 ml of distilled water and 600 ml of ethyl acetate were added for solvent extraction. The organic layer thus obtained was dried over anhydrous magnesium sulfate and distilled off the solvent by a rotary evaporator, to give a yellow liquid.

This liquid obtained was separated by column chromatography using a mixture of ethyl acetate/n-hexane (1:1) as a mobile phase, to obtain 76.5 g of (S)-3-carbamoyl-2-phenyl propanol sulfamates a white solid.
Yield 93%
H-NMR(DMSO, 200MHz), ppm (δ): 3.35(1H,q), 4.16(2H,d), 4.34(2H,d), 6.57(2H,br), 7.39(5H,s), 7.58(2H,s)

### EXAMPLE 75

### Preparation of (S)-3-N-Methylcarbamoyl-2-phenyl propanol sulfamate

Except for using (R)-3-N-methylcarbamoyl-2-phenyl propanol, instead of (R)-3-carbamoyl-2-phenyl propanol, as the starting material, the same procedure with that of Example 74 was repeated.

A concentrated reaction mixture thus obtained was separated by column chromatography using a mixture of ethyl acetate/n-hexane (1:1) as a mobile phase, to obtain 77.8 g of (S)-3-N-methylcarbamoyl-2-phenyl propanol sulfamate as a white solid.
Yield 90%
H-NMR(DMSO), 200MHz), ppm(δ): 2.54(3H,d), 3.35(1H,q), 4.29(4H,m), 6.98(1H,br), 7.29(5H,s), 7.53(2H,s)

### EXAMPLE 76

### Preparation of (S)-3-N,N'-Dimethylcarbamoyl-2-phenyl propanol sulfamate

Except for using (R)-3-N,N'-dimethylcarbamoyl-2-phenyl propanol, instead of (R)-3-carbamoyl-2-phenyl propanol, as the starting material, the same procedure with that of Example 74 was repeated.

A concentrated reaction mixture thus obtained was separated by column chromatography using a mixture of ethyl acetate/n-hexane (1:1) as a mobile phase, to obtain 79.7 g of (S)-3-N,N'-dimethylcarbamoyl-2-phenyl propanol sulfamate as a white solid.
Yield 88%
H-NMR(CDCl₃, 200MHz), ppm(δ): 2.84(6H,d), 3.38(1H,q), 4.39(4H,m), 4.93(2H,s), 7.21(5H,d)

### EXAMPLE 77

### Preparation of (S)-3-N-Isopropyl carbamoyl-2-phenyl propanol sulfamate

Except for using (R)-3-N-isopropyl carbamoyl-2-phenyl propanol, instead of (R)-3-carbamoyl 2-phenyl propanol, as the starting material, the same procedure with that of Example 74 was repeated.

A concentrated reaction mixture thus obtained was separated by column chromatography using a mixture of ethyl acetate/n-hexane (1:1) as a mobile phase, to obtain 84.3 g of (S)-3-N-isopropyl carbamoyl-2-phenyl propanol sulfamate as a white solid.
Yield 89%
H-NMR(DMSO, 200MHz), ppm (δ): 1.01(6H,m), 3.32(1H,m 3.55(1H,q), 4.18(2H,d), 4.29(2H,d), 6.95(1H,d), 7.34(5H,s), 7.56(2H,s)

### EXAMPLE 78

### Preparation of (S)-3-N-Cyclopropyl carbamoyl-2-phenyl propanol sulfamate

Except for using (R)-3-N-cyclopropyl carbamoyl-2-phenyl propanol, instead of (R)-3-carbamoyl-2-phenyl propanol, as the starting material, the same procedure will that of Example 74 was repeated.

A concentrated reaction mixture thus obtained was separated by column chromatography using a mixture of ethyl acetate/n-hexane (1:1) as a mobile phase, to produce 86.7 g of (R)-3-N-cyclopropyl carbamoyl-2-phenyl propanol sulfamate as a white solid.
Yield 92%
H-NMR(DMSO, 200MHz) ppm(δ): 0.35-0.58(4H,m), 2.51(1H,s), 3.35(1H,q), 4.16-4.36(4H,m ), 7.15(1H,hr), 7.29(5H,s), 7.52(2H,s)

### EXAMPLE 79

### Preparation of (S)-3-N-Morphoryl carbamoyl-2-phenyl propanol sulfamate

Except for using (R)-3-N-morphoryl carbamoyl-2-phenyl propanol, instead of (R)-3-carbamoyl-2-phenyl propanol, as the starting material, the same procedure with that of Example 74 was repeated.

The liquid thus obtained was separated by column chromatography using a mixture of ethyl acetate/n-hexane (1:1) as a mobile phase, to produce 94 g of (S)-3-N-morphorylcarbamoyl-2-phenyl propanol sulfamate as a white solid.
Yield 91%
H-NMR(DMSO, 200MHz) ppm(δ): 3.05(1H,q) 3.20-3.50(8H,br), 4.15(4H,m), 4.43(2H,s), 7.13(5H,m)

### EXAMPLE 80

### Preparation of (S)-3-N-Methylcarbamoyl-2-(p-methoxy phenyl) propanol sulfamate

A well dried 2.000 ml flask equipped with a thermometer was purged will nitrogen gas, to completely take off moisture and air from the inside of the flask. This purging was continued for 30 min, after which 1,200 ml of purified acetonitrile was poured into the flask. 71.8 g of (R)-3-N-methylcarbamoyl-2-(p-methoxyphenyl) propanol prepared in Example 73 and 156.8 ml of diisopropyl ethylamine were well dissolved in the solvent and made to be homogeneous by stirring for 30 min. While being maintained at 0°C, the homogeneous solution was slowly added with 69.3 g of sulfamoyl chloride (prepared in the method described in Chem. Ber., 91, 1339-1341 (1958), to Appel and Berger). At the same temperature, the reaction was proceeded and its progress was monitored by thin layer chromatography and liquid chromatography. Roughly, it took about 2 hours to finish the reaction.

After completion of the reaction, acetonitrile remaining was completely removed from the reaction mixture by using a rotary evaporator. 600 ml of distilled water and 600 ml ethyl acetate were added for solvent extraction. The organic layer thus obtained was dried over anhydrous magnesium sulfate and distilled off the solvent by a rotary evaporator, to give a yellow liquid.

This liquid obtained was separated by column chromatography using a mixture of ethyl acetate/n-hexane (1:1) as a mobile phase, to produce 84 g of (S)-3-N-methylcarbamoyl-2-(p-methoxyphenyl) propanol sulfamate as a white solid.
Yield 88%
H-NMR(DMSO), 200 MHz), ppm (δ): 2.45(3H,d), 3.31(1H,q), 4.35(4H,m ), 6.48(1H,br), 6.89(2H,d), 7.49(2H,s)

The compounds according to the present invention are very useful for the prophylaxis and treatment of CNS disorder, for example, nervous myalgia, epilepsy and minimal brain dysfunction.

The present invention has been described in an illustrative manner, and it is to be understood the terminology used is intended to be in the nature of description rather than of limitation.

Many modifications and variations of the present invention are possible in light of the above teachings. Therefore, it is to be understood that within the scope of the appended claims, the invention may be practiced otherwise than as specifically described.

## Claims

1. N-N'-substituted carbamoyl-2-aryl propanol sulfamate racemates, represented by the following formula I: 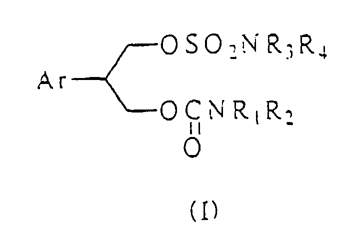 wherein,
Ar is represented by the following formulas; 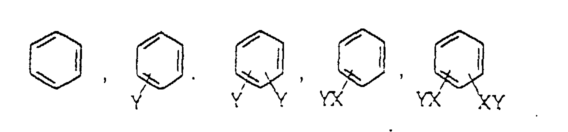
Y is selected from the group consisting of halogens such as F, Cl, Br and I, trifluoromethyl and alkyl groups containing 1 to 3 carbon atoms when Y alone binds to benzene ring and from the group consisting of trifluoromethyl and alkyl groups containing 1 to 3 carbon atoms when Y binds to X which is O or S; and
R₁, R₂, R₃ and R₄, identical or different, each are selected from the group consisting of hydrogen, linear or branched alkyl groups containing 1 to 16 carbon atoms, cyclic alkyl groups containing 3 to 16 carbon atoms and aryl groups containing 6 to 8 carbon atoms, and NR₁R₂ and NR₃R₄, identical or different, each may form a 3 to 7-membered aliphatic cyclic compound together with another nitrogen atom or oxygen atom.

2. (R)-N,N' - substituted carbamoyl-2-aryl propanol sulfamates, represented by the following formula II : 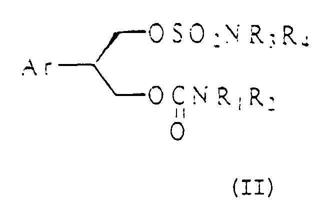 wherein, Ar, R₁, R₂, R₃ and R₄ have the meaning according to claim 1.

3. (S)-N,N' - substituted carbamoyl-2-aryl propanol sulfamates, represented by the following formula III : 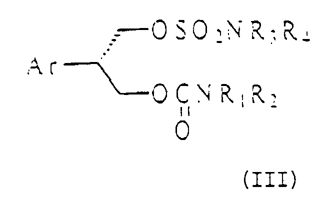 wherein, Ar, R₁, R₂, R₃ and R₄ have the meaning according to claim 1.

4. 3-N - substituted carbamoyl-2-aryl propanol acetate represented by the following formula IV : 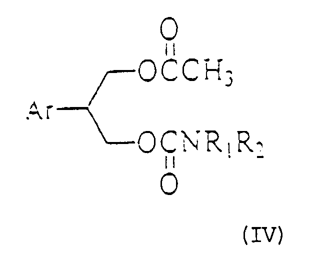 wherein, Ar, R₁ and R₂ have the meaning according to claim 1.

5. 3-N - substituted carbamoyl-2-aryl propanol, represented by the following formula V : 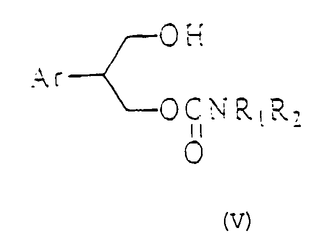 Wherein, Ar, R₁ and R₂ have the meaning according to claim 1.

6. 3-Acetoxy-2-aryl propanol sulfamate, represented by the following formula VI : 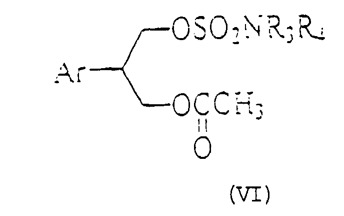 wherein, Ar, R₃ and R₄ have the meaning according to claim 1.

7. 2-Aryl-1,3- propandiol monosulfamate, represented by the following formula VII : 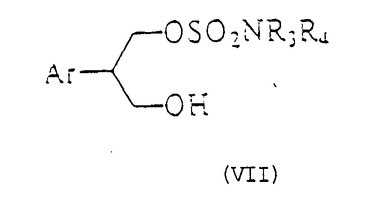 wherein Ar, R₃ and R₄ have the meaning according to claim 1.

8. (S)-3-N-substituted carbamoyl-2-aryl propanol acetate, represented by the following formula VIII 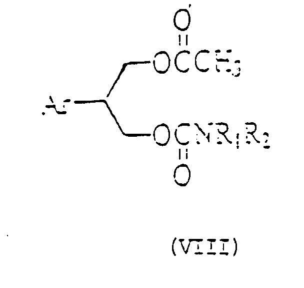 wherein, Ar, R₁ and R₂ have the meaning according to claim 1, with the proviso that R₁ and R₂ are not simultaneously a hydrogen atom.

9. (S)-3-N-substituted carbamoyl-2-aryl propanol, represented by the following formula IX : 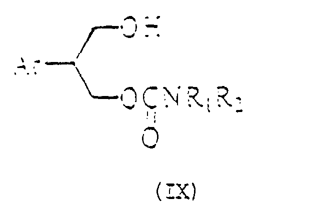 wherein, Ar, R₁ and R₂ have the meaning according to claim 1, with the proviso that R₁ and R₂ are not simultaneously a hydrogen atom.

10. (S)-3-acetoxy-2-aryl-1,3-propandiol sulfamate, represented by the following formula X : 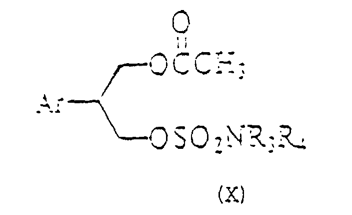 wherein, Ar, R₃ and R₄ have the meaning according to claim 1.

11. (S)-2-aryl-1,3-propandiol monosulfamate, represented by the following formula XI : 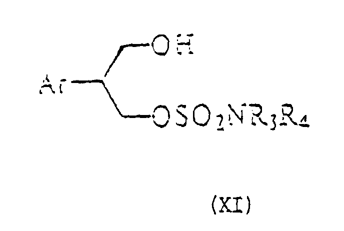 wherein, Ar, R₃ and R₄ have the meaning according to claim 1.

12. R-3-N-substituted carbamoyl-2-aryl propanol acetate, represented by the following formula XII : 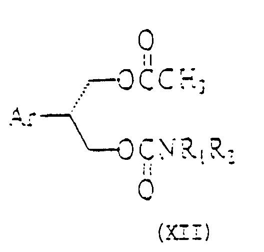 wherein, Ar, R₁ and R₂ have the meaning according to claim 1, with the proviso that R₁ and R₂ are not simultaneously a hydrogen atom.

13. R-3-N-substituted carbamoyl-2-aryl propanol, represented by the following formula XIII : 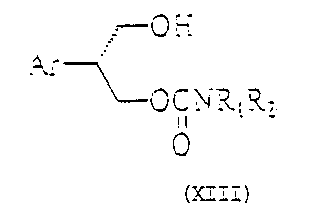 wherein, Ar, R₁ and R₂ have the meaning according to claim 1, with the proviso that R₁ and R₂ are not simultaneously a hydrogen atom.

14. A method for preparing an N,N'-substituted carbamoyl-2-aryl propanol sulfamate racemate, represented by the following formula I which comprises reacting 3-acetoxy-2-aryl propanol, represented by the following formula XIV, with carbonyl diimidazole to give 3-imidazolyl carbonyloxy-2-aryl propanol acetate, represented by the following formula XV, reacting the 3-imidazolyl carbonyloxy-2-aryl propanol acetate with a substituted amine, represented by the following formula XVI, to give a 3-N-substituted carbamoyl-2-aryl propanol acetate, represented by the following formula IV, subjecting the 3-N-substituted carbamoyl-2-aryl propanol acetate to transesterification in an alcohol solvent in the presence or a base catalyst, to produce a 3-N-substituted carbamoyl-2-aryl propanol, represented by the following formula V, and reacting the 3-N-substituted carbamoyl-2-aryl propanol with sulfamoyl chloride: 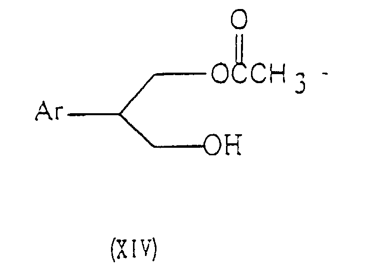 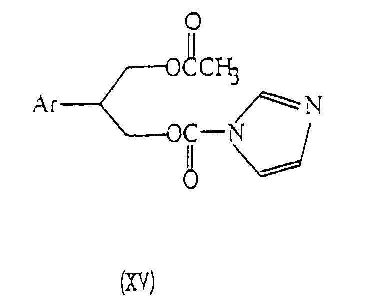
HNR₁R₂ (XVI) 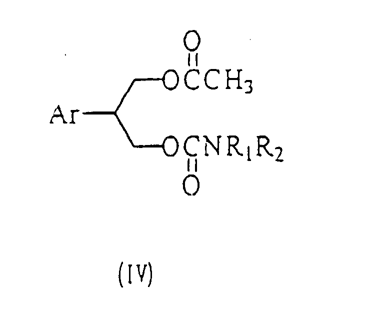 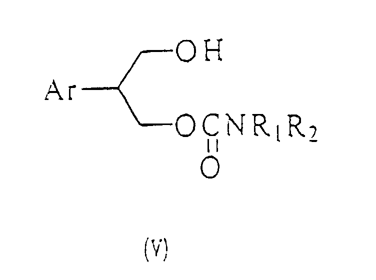 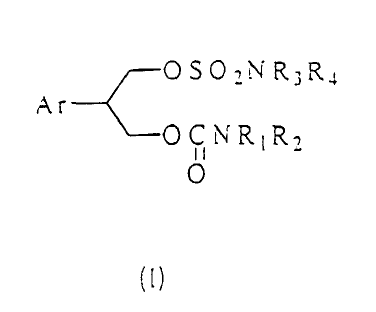
wherein,
Ar is represented by the following formulas; 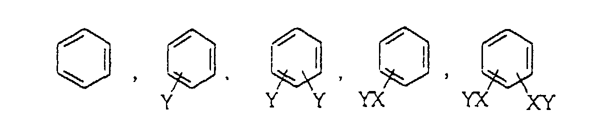
Y is selected from the group consisting of halogens such as F, Cl, Br and I, trifluoromethyl and alkyl groups containing 1 to 3 carbon atoms when Y alone binds to benzene ring and from the group consisting of trifluoromethyl and alkyl groups containing 1 to 3 cabon atoms when Y binds to X which is O or S; and
R₁, R₂, R₃ and R₄, identical or different, each are selected from the group consisting of hydrogen, linear or branched alkyl groups containing 1 to 16 carbon atoms, cyclic alkyl groups containing 3 to 16 carbon atoms and aryl groups containing 6 to 8 carbon atoms, and NR₁R₂ and NR₃R₄, identical or different, each may form a 3 to 7-membered aliphatic cyclic compound together with another nitrogen atom or oxygen atom.

15. A method for preparing an N,N'-substituted carbamoyl-2-aryl propanol sulfamate, represented by the following formula I, which comprises reacting 3-acetoxy-2-aryl propanol, represented by the following formula XIV, with sulfamoyl chloride in the presence of a base catalyst, to produce a 3-acetoxy-2-aryl propanol sulfamate, represented by following formula VI, subjecting the 3-acetoxy-2-aryl propanol sulfamate to transesterification in an alcohol solvent in the presence of a base catalyst, to produce a 2-aryl-1,3-propandiol monosulfamate, represented by the following formula VII, reacting the 2-aryl-1,3-propandiol monosulfamate with carbonyl diimidazole to give a 3-imidazolyl carbonyloxy-2-aryl propanol sulfamate, represented by the following formula XVII, and substituting the imidazole group of the 3-imidazolyl carbonyloxy-2-aryl propanol 'sulfamate with a substituted amine, represented by the following formula XVII: 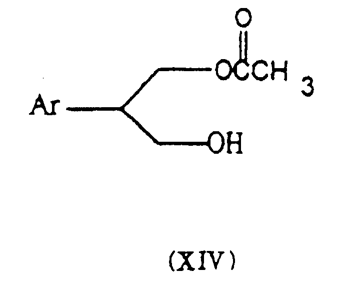 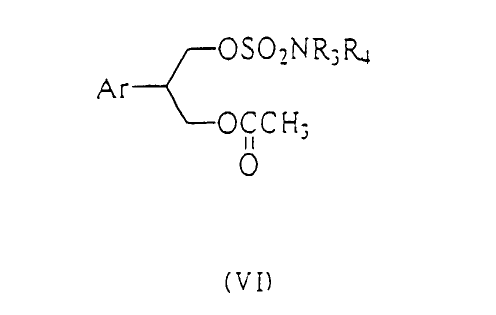 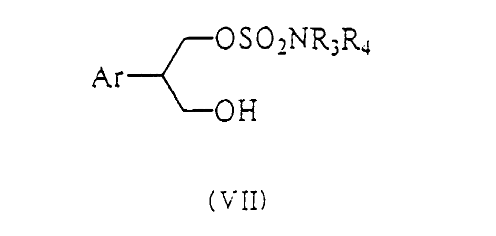 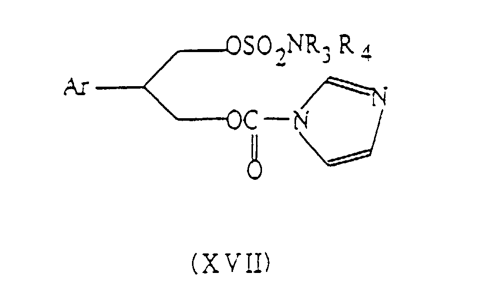
HNR₁R₂ (XVI) 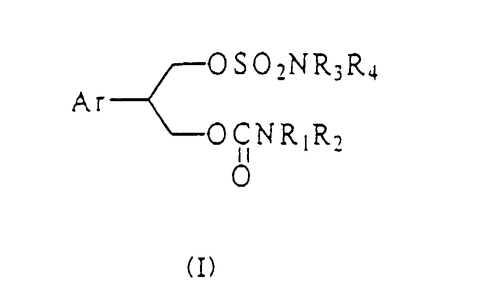
wherein, Ar, R₁, R₂, R₃ and R₄ have the meaning according to claim 1.

16. A method for preparing an N,N'-substituted carbamoyl-2-aryl-1,3-propandiol sulfamate, represented by the following formula I, which comprises reacting a 2-aryl-1,3-propandiol monosulfamate represented by the following formula VII, with an isocyanate represented by the following formula XVIII: 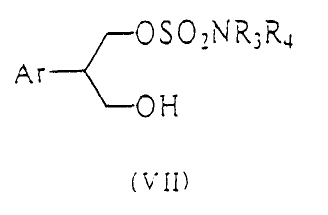
R₁NCO (XVIII) 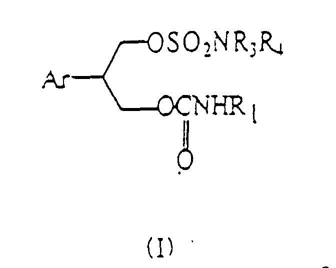
wherein, Ar, R₁, R₃ and R₄ have the meaning according to claim 1.

17. A method for preparing an R-N,N'-substituted carbamoyl-2-aryl propanol sulfamate represented by the following formula II which comprises reacting (R)-3-acetoxy-2-aryl propanol, represented by the following formula XIX, with carbonyl diimidazole to give an (S)-3-imidazolyl carbonyloxy-2-aryl propanol acetate represented by the following formula XX, substituting the imidazole group of the (S)-3-imidazolyl carbonyloxy-2-aryl propanol acetate with a suhstituted amine, represented by the following formula XVI, to give an (S)-3-N-substituted carbamoyl-2-aryl propanol acetate, represented by the following formula VIII, subjecting the (S)-3-N-substituted carbamoyl-2-aryl propanol acetate to transesterification in an alcohol solvent in the presence of a base catalyst, to produce an (S)-3-N-substituted carbamoyl-2-aryl propanol, represented by the following formula IX, and reacting the (S)-3-N-substituted carbamoyl-2-aryl propanol with sulfamoyl chloride: 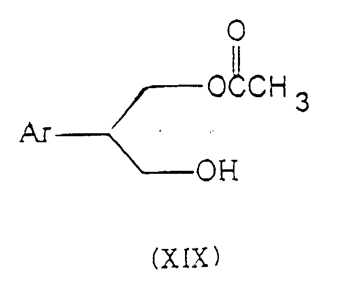 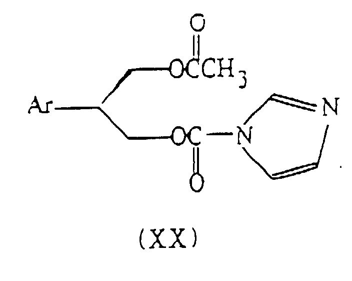
HNR₁R₂ (XVI) 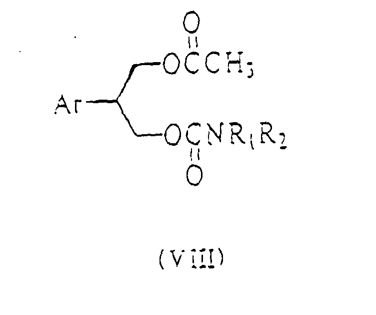 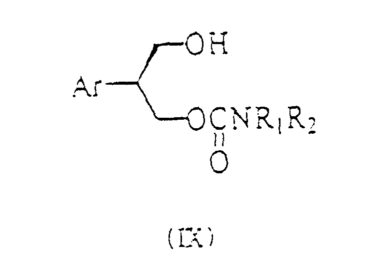 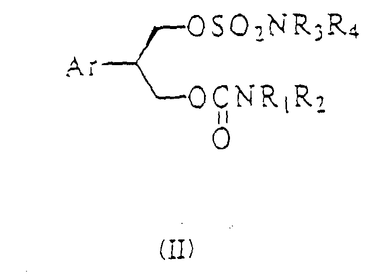
wherein, Ar, R₁, R₂, R₃ and R₄ have the meaning according to claim 1.

18. A method for preparing an (S)-N,N'-substituted carbamoyl-2-aryl propanol sulfamate, represented by the following formula III, which comprises reacting an (R)-3-acetoxy-2-aryl propannl, represented by the following formula XIX, with sulfamoyl chloride in the presence of a base catalyst, to produce an (S)-3-acetoxy-2-aryl-1,3-propandiol sulfamate, represented by following formula X, subjecting the (S)-3-acetoxy-2-aryl-1,3-propandiol sulfamate to transesterification in an alcohol solvent in the presence of a base catalyst, to produce an (S)-2-aryl-1,3-propandiol monosulfamate, represented by the following formula XI, reacting the (S)-2-aryl-1,3-propandiol monosulfamate with carbonyl diimidazole to give an (S)-3-imidazolyl carbonyloxy-2-aryl propanol sulfamate, represented by the following formula XXI, and substituting the imidazole group of the (S)-3-imidazolyl carbonyloxy-2-aryl propanol sulfamate with a substituted amine, represented by the following formula XVI: 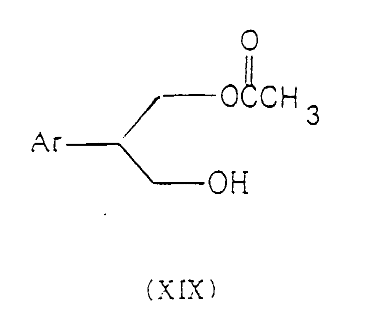 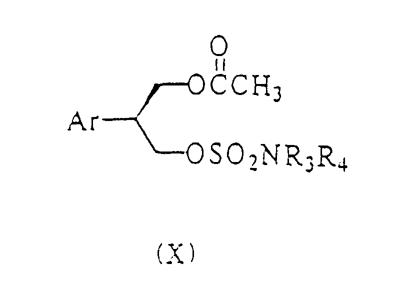 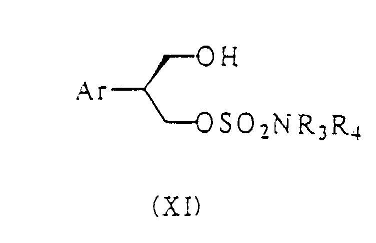 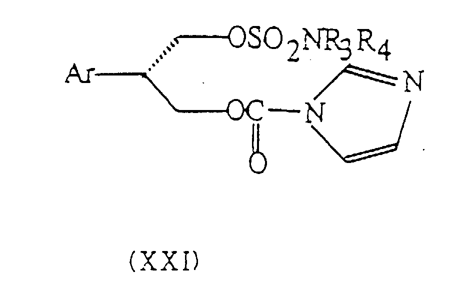
HNR₁R₂ (XVI) 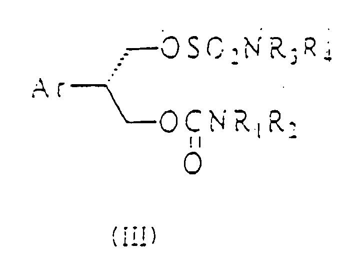
wherein, Ar, R₁, R₂, R₃ and R₄ have the meaning according to claim 1.

19. A method for preparing an (S)-N,N'-substituted carbamoyl-2-aryl propanol sulfamate, represented by the following formula III, which comprises reacting an (S)-3-acetoxy-2-aryl propanol represented by the following formula XXII, with carbonyl diimidazole to give an (R)-3-imidazolyl carbonyloxy-2-aryl propanol acetate, represented by the following formula XXIII, substituting the imidazole group of the (R)-3-imidazolyl carbonyloxy-2-aryl propanol acetate with a subsituted amine, represented by the following formula XVI, to give an (R)-3-N-substituted carbamoyl-2-aryl propanol acetate, represented by the following formula XII, subjecting the (R)-3-N-substituted carbamoyl-2-aryl propanol acetate to transesterification in an alcohol solvent in the presence of a base catalyst, to produce an (R)-3-N-substituted carbamoyl-2-aryl propanol, represented by the following formula XIII, and reacting the (R)-3-N-substituted carbamoyl-2-aryl propanol with sulfamoyl chloride: 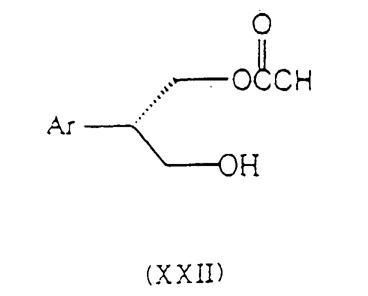 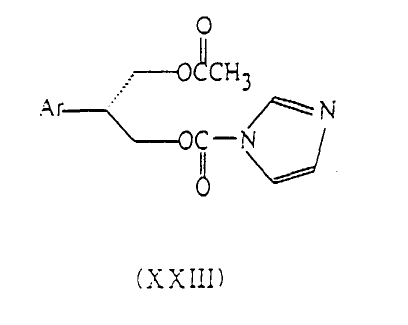
HNR₁R₂ (XVI) 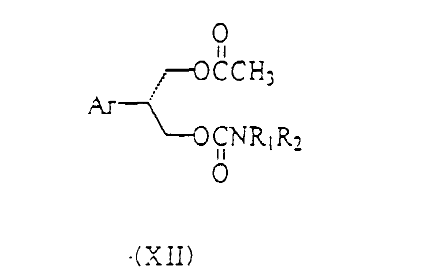 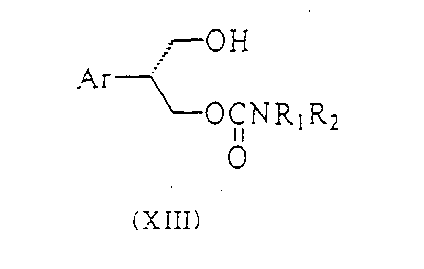 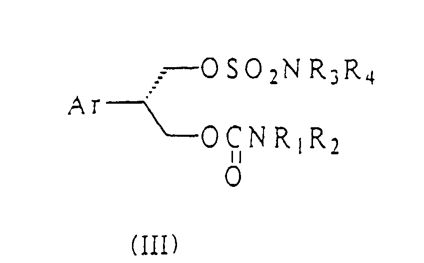
wherein, Ar, R₁, R₂, R₃ and R₄ have the meaning according to claim 1.

20. A pharmaceutical composition for treating disorders of the central nervous system which comprises as an active ingredient an effective amount for treating disorders of the central nervous system of a compound of Formula (I) as defined in claim 1, Formula (II) as defined in claim 2, or Formula (III) as defined in claim 3 and a pharmaceutically acceptable carrier.

21. The composition of claim 20, wherein the CNS disorder being treated is selected from the group consisting of convulsions or epilepsy.

## Patentansprüche

1. N,N'-substituierte Carbamoyl-2-arylpropanolsulfamat-Racemate, dargestellt durch die folgende Formel I: 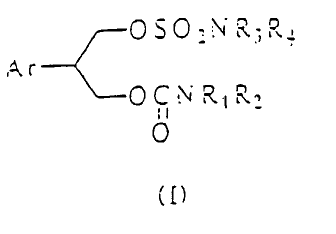 wobei
Ar für die folgenden Formeln steht: 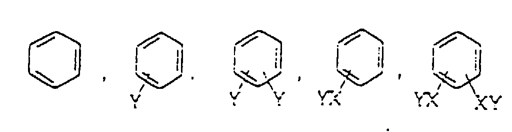
Y ausgewählt wird aus der Gruppe, die besteht aus Halogenen, beispielsweise F, Cl, Br und I, Trifluormethyl und Alkylgruppen, die 1 bis 3 Kohlenstoffatome enthalten, wenn Y allein an einen Benzolring gebunden ist, und aus der Gruppe, die besteht aus Trifluormethyl und Alkylgruppen, die 1 bis 3 Kohlenstoffatome enthalten, wenn Y an X, was O oder S ist, gebunden ist, und
R₁, R₂, R₃ und R₄, die identisch oder verschieden sind, jeweils aus der Gruppe ausgewählt werden, die besteht aus Wasserstoff, linearen oder verzweigten Alkylgruppen, die 1 bis 16 Kohlenstoffatome enthalten, cyclischen Alkylgruppen, die 3 bis 16 Kohlenstoffatome enthalten, und Arylgruppen, die 6 bis 8 Kohlenstoffatome enthalten, und NR₁R₂ und NR₃R₄, die identisch oder verschieden sind, jeweils eine 3- bis 7-gliedrige aliphatische cyclische Verbindung zusammen mit einem weiteren Stickstoffatom oder Sauerstoffatom bilden können.

2. (R)-N,N'-substituierte Carbamoyl-2-arylpropanolsulfamate, dargestellt durch die folgende Formel II: 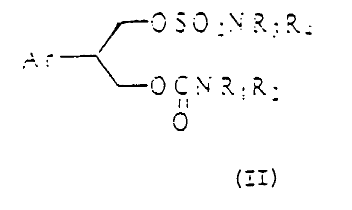 wobei Ar, R₁, R₂, R₃ und R₄ die Bedeutung gemäß Anspruch 1 haben.

3. (S)-N,N'-substituierte Carbamoyl-2-arylpropanolsulfamate, dargestellt durch die folgende Formel III: 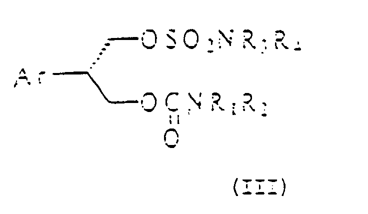 wobei Ar, R₁, R₂, R₃ und R₄ die Bedeutung gemäß Anspruch 1 haben.

4. 3-N-substituiertes Carbamoyl-2-arylpropanolacetat, dargestellt durch die folgende Formel IV: 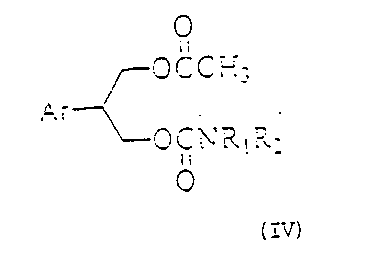 wobei Ar, R₁ und R₂ die Bedeutung gemäß Anspruch 1 haben.

5. 3-N-substituiertes Carbamoyl-2-arylpropanol, dargestellt durch die folgende Formel V: 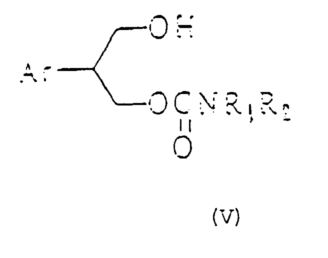 wobei Ar, R₁ und R₂ die Bedeutung gemäß Anspruch 1 haben.

6. 3-Acetoxy-2-arylpropanolsulfamat, dargestellt durch die folgende Formel VI: 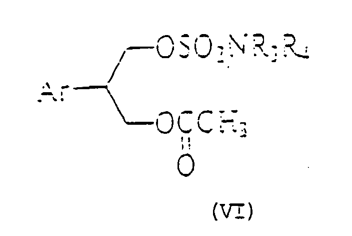 wobei Ar, R₃ und R₄ die Bedeutung gemäß Anspruch 1 haben.

7. 2-Aryl-1,3-propandiolmonosulfamat, dargestellt durch die folgende Formel VII: 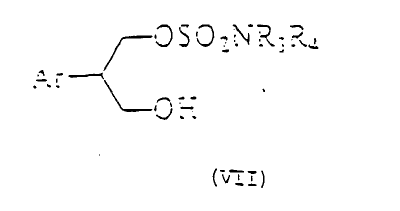 wobei Ar, R₃ und R₄ die Bedeutung gemäß Anspruch 1 haben.

8. (S)-3-N-substituiertes Carbamoyl-2-arylpropanolacetat, dargestellt durch die folgende Formel VIII: 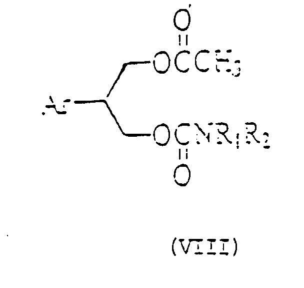 wobei Ar, R₁ und R₂ die Bedeutung gemäß Anspruch 1 haben.

9. (S)-3-N-substituiertes Carbamoyl-2-arylpropanol, dargestellt durch die folgende Formel IX: 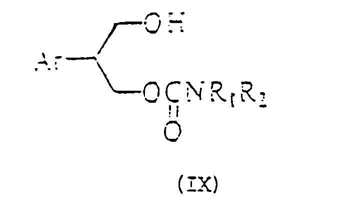 wobei Ar, R₁ und R₂ die Bedeutung gemäß Anspruch 1 haben.

10. (S)-2-Acetoxy-2-aryl-1,3-propandiolsulfamat, dargestellt durch die folgende Formel X: 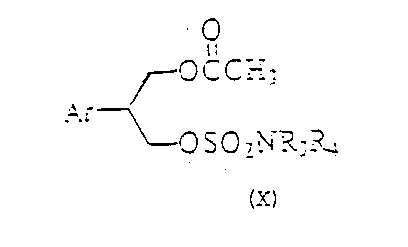 wobei Ar, R₃ und R₄ die Bedeutung gemäß Anspruch 1 haben.

11. (S)-2-Aryl-1,3-propandiolmonosulfamat, dargestellt durch die folgende Formel XI: 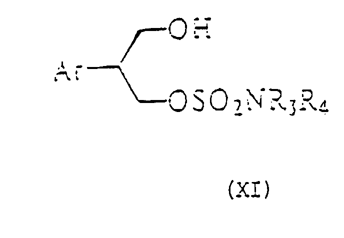 wobei Ar, R₃ und R₄ die Bedeutung gemäß Anspruch 1 haben.

12. R-3-N-substituiertes Carbamoyl-2-arylpropanolacetat, dargestellt durch die folgende Formel XII: 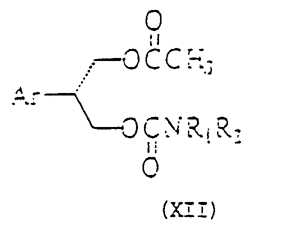 wobei Ar, R₁ und R₂ die Bedeutung gemäß Anspruch 1 haben.

13. R-3-N-substituiertes Carbamoyl-2-arylpropanol, dargestellt durch die folgende Formel XIII: 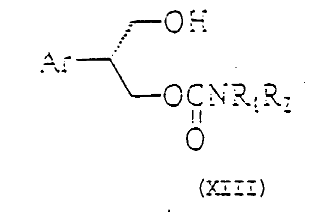 wobei Ar, R₁ und R₂ die Bedeutung gemäß Anspruch 1 haben.

14. Verfahren zur Herstellung von N,N'-substituiertem Carbamoyl-2-arylpropanolsulfamat-Racemat, dargestellt durch die folgende Formel I, was umfaßt ein Umsetzen von 3-Acetoxy-2-arylpropanol, dargestellt durch die folgende Formel XIV, mit Carbonyldiimidazol, um 3-Imidazolylcarbonyloxy-2-arylpropanolacetat, dargestellt durch die folgende Formel XV, zu ergeben, Umsetzen des 3-Imidazolylcarbonyloxy-2-arylpropanolacetats mit substituiertem Amin, dargestellt durch die folgende Formel XVI, um 3-N-substituiertes Carbamoyl-2-arylpropanolacetat, dargestellt durch die folgende Formel IV, zu ergeben, Unterwerfen des 3-N-substituierten Carbamoyl-2-arylpropanolacetats einer Umesterung in einem Alkohol-Lösungsmittel in Gegenwart eines basischen Katalysators, um 3-N-substituiertes Carbamoyl-2-arylpropanol, dargestellt durch die folgende Formel V, herzustellen, und Umsetzen des 3-N-substituierten Carbamoyl-2-arylpropanols mit Sulfamoylchlorid: 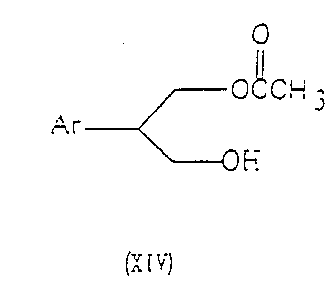 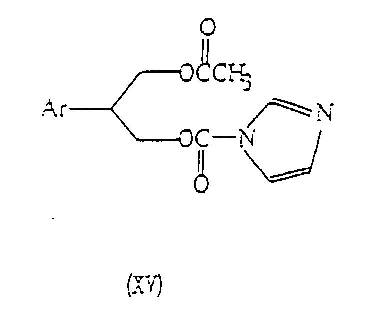
HNR₁R₂ (XVI) 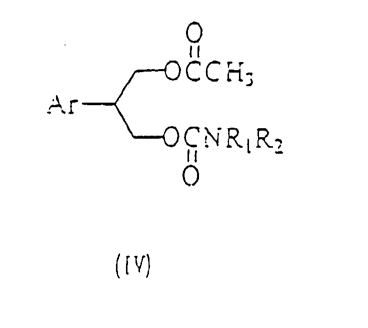 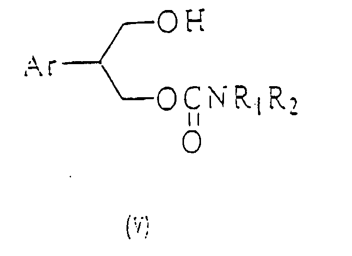 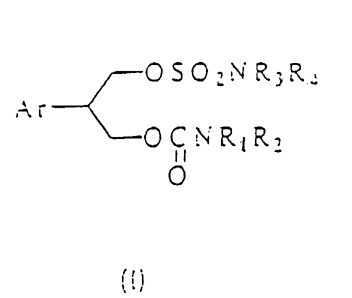
wobei
Ar für die folgenden Formeln steht: 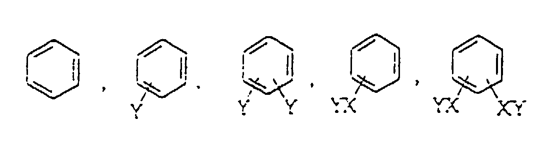
Y ausgewählt wird aus der Gruppe, die besteht aus Halogenen, beispielsweise F, Cl, Br und I, Trifluormethyl und Alkylgruppen, die 1 bis 3 Kohlenstoffatome enthalten, wenn Y allein an einen Benzolring gebunden ist, und aus der Gruppe, die besteht aus Trifluormethyl und Alkylgruppen, die 1 bis 3 Kohlenstoffatome enthalten, wenn Y an X, was O oder S ist, gebunden ist, und
R₁, R₂, R₃ und R₄, die identisch oder verschieden sind, jeweils aus der Gruppe ausgewählt werden, die besteht aus Wasserstoff, linearen oder verzweigten Alkylgruppen, die 1 bis 16 Kohlenstoffatome enthalten, cyclischen Alkylgruppen, die 3 bis 16 Kohlenstoffatome enthalten, und Arylgruppen, die 6 bis 8 Kohlenstoffatome enthalten, und NR₁R₂ und NR₃R₄, die identisch oder verschieden sind, jeweils eine 3-bis 7-gliedrige aliphatische cyclische Verbindung zusammen mit einem weiteren Stickstoffatom oder Sauerstoffatom bilden können.

15. Verfahren zur Herstellung von N,N'-substituiertem Carbamoyl-2-arylpropanolsulfamat, dargestellt durch die folgende Formel I, was umfaßt ein Umsetzen von 3-Acetoxy-2-arylpropanol, dargestellt durch die folgende Formel XIV, mit Sulfamoylchlorid in Gegenwart eines basischen Katalyators, um 3-Acetoxy-2-arylpropanolsulfamat, dargestellt durch die folgende Formel VI, herzustellen, Unterwerfen des 3-Acetoxy-2-arylpropanolsulfamats einer Umesterung in einem Alkohol-Lösungsmittel in Gegenwart eines basischen Katalysators, um 2-Aryl-1,3-propandiolmonosulfamat, dargestellt durch die folgende Formel VII, herzustellen, Umsetzen des 2-Aryl-1,3-propandiolmonosulfamats mit Carbonyldiimidazol, um 3-Imidazolylcarbonyloxy-2-arylpropanolsulfamat, dargestellt durch die folgende Formel XVII, zu ergeben, und Substituieren der Imidazolgruppe des 3-Imidazolylcarbonyloxy-2-arylpropanolsulfamats mit substituiertem Amin, dargestellt durch die folgende Formel XVI: 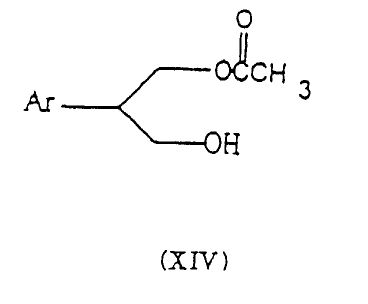 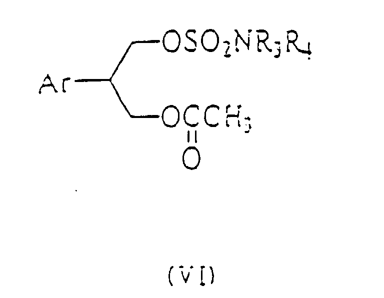 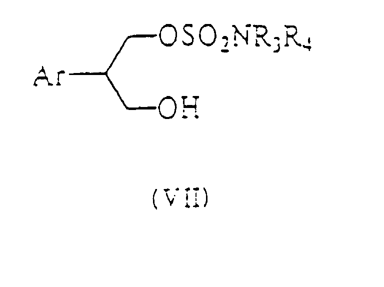 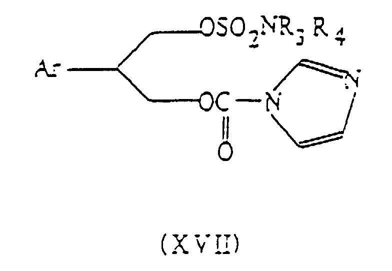
HNR₁R₂ (XVI) 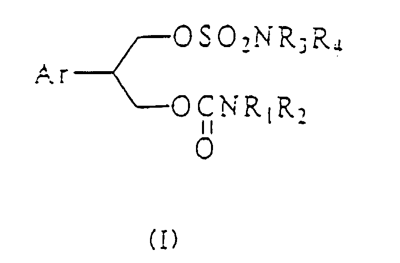
wobei Ar, R₁, R₂, R₃ und R₄ die Bedeutung gemäß Anspruch 1 haben.

16. Verfahren zur Herstellung von N,N'-substituiertem Carbamoyl-2-aryl-1,3-propandiolsulfamat, dargestellt durch die folgende Formel I, was umfaßt ein Umsetzen von 2-Aryl-1,3-propandiolmonosulfamat, dargestellt durch die folgende Formel VII, mit einem Isocyanat, dargestellt durch die folgende Formel XVIII: 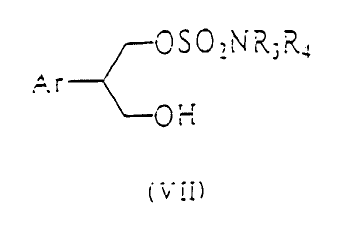
R₁NCO (XVIII) 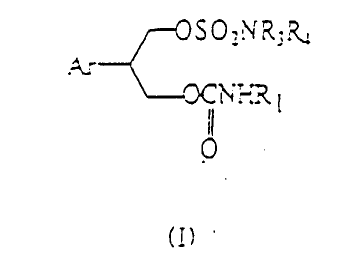
wobei Ar, R₁, R₃ und R₄ die Bedeutung gemäß Anspruch 1 haben.

17. Verfahren zur Herstellung von R-N,N'-substituiertem Carbamoyl-2-arylpropanolsulfamat, dargestellt durch die folgende Formel II, was umfaßt ein Umsetzen von (R)-3-Acetoxy-2-arylpropanol, dargestellt durch die folgende Formel XIX, mit Carbonyldiimidazol, um (S)-3-Imidazolylcarbonyloxy-2-arylpropanolacetat, dargestellt durch die folgende Formel XX, zu ergeben, Substituieren der Imidazolgruppe des (S)-3-Imidazolylcarbonyloxy-2-arylpropanolacetats mit substituiertem Amin, dargestellt durch die folgende Formel XVI, um (S)-3-N-substituiertes Carbamoyl-2-arylpropanolacetat, dargestellt durch die folgende Formel VIII, zu ergeben, Unterwerfen des (S)-3-N-substituierten Carbamoyl-2-arylpropanolacetats einer Umesterung in einem Alkohol-Lösungsmittel in Gegenwart eines basischen Katalysators, um (S)-3-N-substituiertes Carbamoyl-2-arylpropanol, dargestellt durch die folgende Formel IX, herzustellen, und Umsetzen des (S)-3-N-substituierten Carbamoyl-2-arylpropanols mit Sulfamoylchlorid: 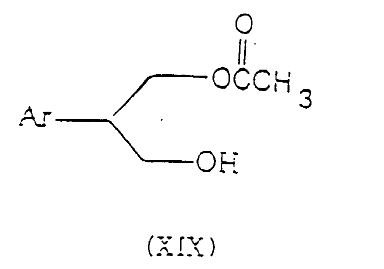 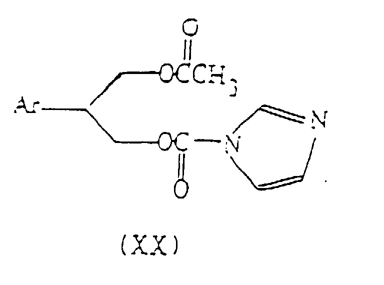
HNR₁R₂ (XVI) 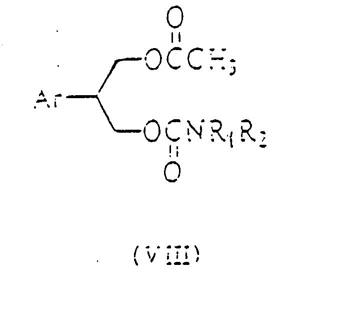 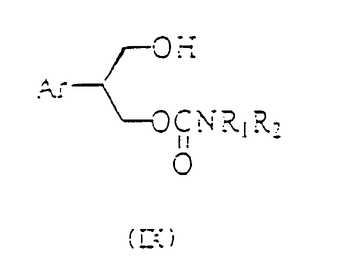 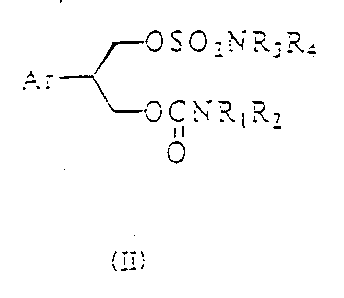
wobei Ar, R₁, R₂, R₃ und R₄ die Bedeutung gemäß Anspruch 1 haben.

18. Verfahren zur Herstellung von (S)-N,N'-substituiertem Carbamoyl-2-arylpropanolsulfamat, dargestellt durch die folgende Formel III, was umfaßt ein Umsetzen von (R)-3-Acetoxy-2-arylpropanol, dargestellt durch die folgende Formel XIX, mit Sulfamoylchlorid in Gegenwart eines basischen Katalysators, um (S)-3-Acetoxy-2-aryl-1,3-propandiolsulfamat, dargestellt durch die folgende Formel X, herzustellen, Unterwerfen des (S)-3-Acetoxy-2-aryl-1,3-propandiolsulfamats einer Umesterung in einem Alkohol-Lösungsmittel in Gegenwart eines basischen Katalyators, um (S)-2-Aryl-1,3-propandiolmonosulfamat, dargestellt durch die folgende Formel XI, herzustellen, Umsetzen des (S)-2-Aryl-1,3-propandiolmonosulfamats mit Carbonyldiimidazol, um (S)-3-Imidazolylcarbonyloxy-2-arylpropanolsulfamat, dargestellt durch die folgende Formel XXI, zu ergeben, und Substituieren der Imidazolgruppe des (S)-3-Imidazolylcarbonyloxy-2-arylpropanolsulfamats mit substituiertem Amin, dargestellt durch die folgende Formel XVI: 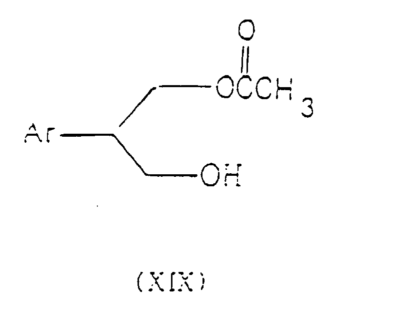 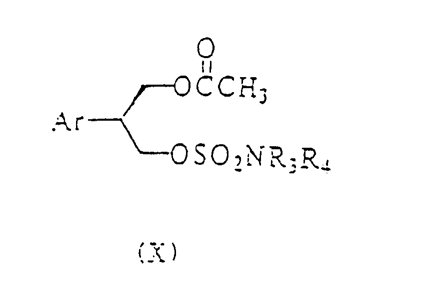 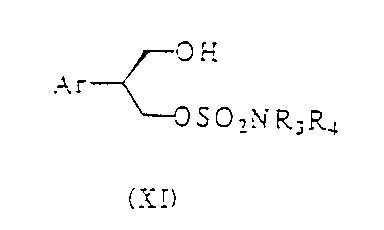 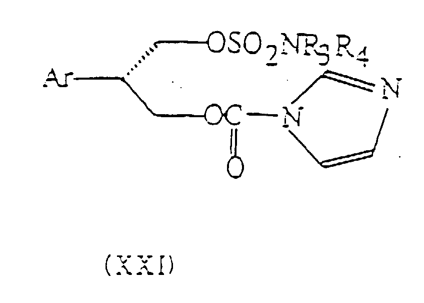
HNR₁R₂ (XVI) 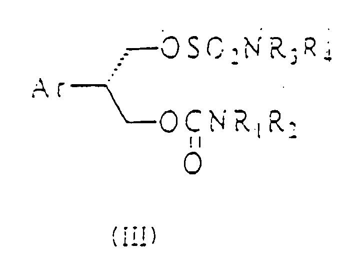
wobei Ar, R₁, R₂, R₃ und R₄ die Bedeutung gemäß Anspruch 1 haben.

19. Verfahren zur Herstellung von (S)-N,N'-substituiertem Carbamoyl-2-arylpropanolsulfamat, dargestellt durch die folgende Formel III, was umfaßt ein Umsetzen von (S)-3-Acetoxy-2-arylpropanol, dargestellt durch die folgende Formel XXII, mit Carbonyldiimidazol, um (R)-3-Imidazolylcarbonyloxy-2-arylpropanolacetat, dargestellt durch die folgende Formel XXIII, zu ergeben, Substituieren der Imidazolgruppe des (R)-3-Imidazolylcarbonyloxy-2-arylpropanolacetats mit substituiertem Amin, dargestellt durch die folgende Formel XVI, um (R)-3-N-substituiertes Carbamoyl-2-arylpropanolacetat, dargestellt durch die folgende Formel XII, zu ergeben, Unterwerfen des (R)-3-N-substituierten Carbamoyl-2-arylpropanolacetats einer Umesterung in einem Alkohol-Lösungsmittel in Gegenwart eines basischen Katalysators, um (R)-3-N-substituiertes Carbamoyl-2-arylpropanol, dargestellt durch die folgende Formel XIII, herzustellen, und Umsetzen des (R)-3-N-substituierten Carbamoyl-2-arylpropanols mit Sulfamoylchlorid: 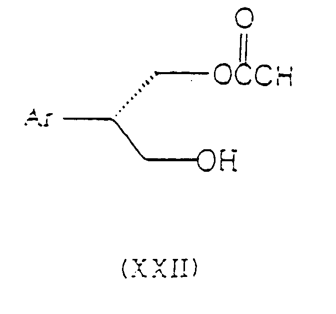 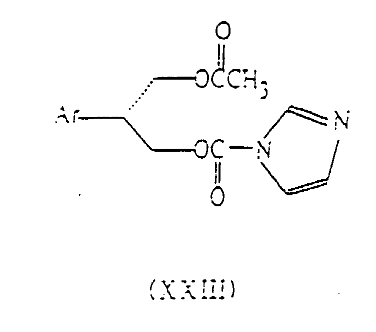
HNR₁R₂ (XVI) 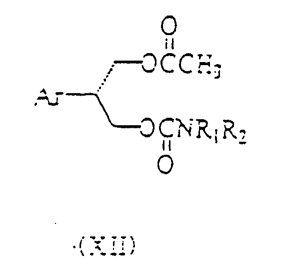 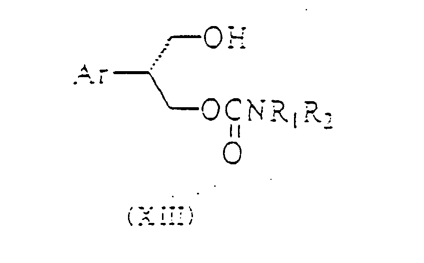 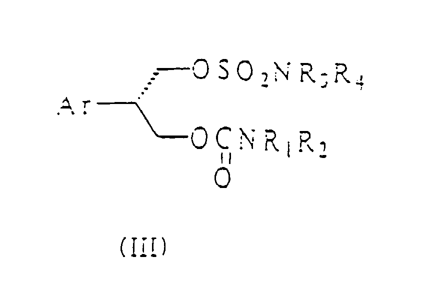
wobei Ar, R₁, R₂, R₃ und R₄ die Bedeutung gemäß Anspruch 1 haben.

20. Pharmazeutische Zusammensetzung zum Behandeln von Fehlfunktionen des Zentralnervensystems, die umfaßt als aktiven Bestandteil eine wirksame Menge zum Behandeln von Fehlfunktionen des Zentralnervensystems einer Verbindung gemäß Formel (I), wie in Anspruch 1 definiert, Formel (II), wie in Anspruch 2 definiert, oder Formel (III), wie in Anspruch 3 definiert, und einen pharmazeutisch annehmbaren Träger.

21. Zusammensetzung gemäß Anspruch 20, wobei die ZNS-Fehlfunktion, die behandelt wird, aus der Gruppe ausgewählt wird, die aus Konvulsionen oder Epilepsie besteht.

## Revendications

1. Racémates de sulfamates de carbamoyl-N,N'-substitué-2-aryl-propanol, représentés par la formule I ci-après : 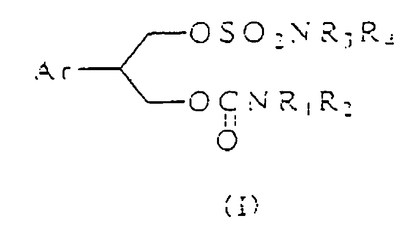 dans laquelle
Ar est représenté par les formules suivantes : 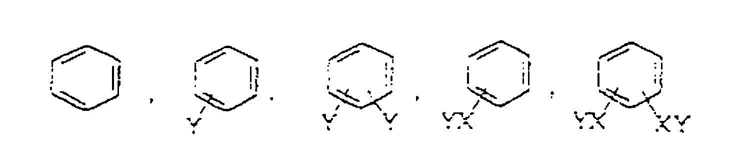
Y est choisi parmi le groupe comprenant les halogènes comme F, Cl, Br et I, les groupes trifluorométhyle et alkyle renfermant 1 à 3 atomes de carbone lorsque Y seul est fixé au noyau de benzène et le groupe comprenant les groupes trifluorométhyle et alkyle renfermant de 1 à 3 atomes de carbone lorsque Y est fixé à X qui est 0 ou S ; et
R₁, R₂, R₃ et R₄, qui sont identiques ou différents, sont chacun choisis parmi le groupe comprenant l'hydrogène, les groupes alkyle linéaires ou ramifiés renfermant de 1 à 16 atomes de carbone, les radicaux alkyle cycliques renfermant de 3 à 16 atomes de carbone et les groupes aryle renfermant de 6 à 8 atomes de carbone, et NR₁R₂ et NR₃R₄, qui sont identiques ou différents, peuvent former chacun un chacun un composé cyclique aliphatique de 3 à 7 membres, conjointement avec un autre atome d'azote ou atome d'oxygène.

2. Sulfamates de carbamoyl (R)-N,N'-substitué-2-aryl-propanol, représentés par la formule suivante II : 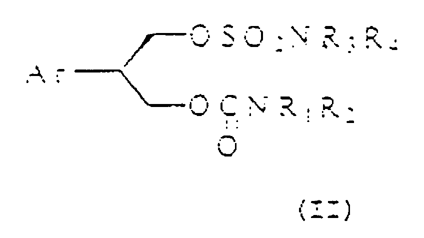 dans laquelle Ar, R₁, R₂, R₃ et R₄ ont la signification selon la revendication 1.

3. Sulfamates de carbamoyl (S)-N,N'-substitué-2-aryl-propanol, représentés par la formule suivante III : 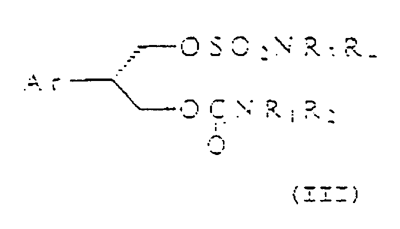 dans laquelle Ar, R₁, R₂, R₃ et R₄ ont la signification selon la revendication 1.

4. Acétate de carbamoyl 3-N-substitué-2-aryl-propanol, représenté par la formule suivante IV : 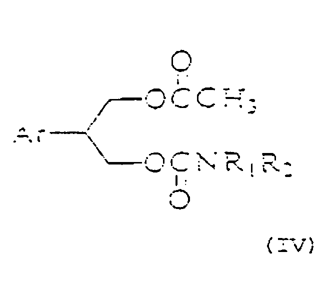 dans laquelle Ar, R₁ et R₂ ont la signification selon la revendication 1.

5. Carbamoyl 3-N-substitué-2-aryl-propanol, représenté par la formule suivante V : 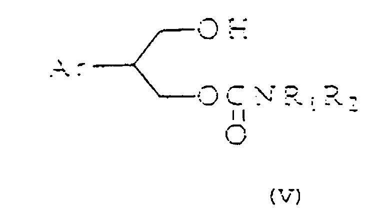 dans laquelle Ar, R₁ et R₂ ont la signification selon la revendication 1.

6. Sulfamate de 3-acétoxy-2-aryl-propanol, représenté par la formule suivante VI : 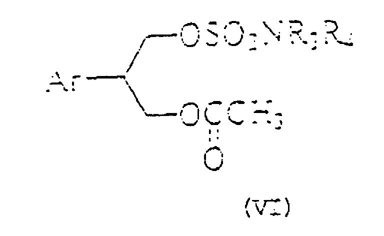 dans laquelle Ar, R₃ et R₄ ont la signification selon la revendication 1.

7. Monosulfamate de 2-aryl-l,3-propanediol, représenté par la formule suivante VII : 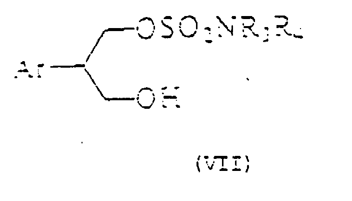 dans laquelle Ar, R₃ et R₄ ont la signification selon la revendication 1.

8. Acétate de carbamoyl (S)-3-N-substitué-2-aryl-propanol, représenté par la formule suivante VIII : 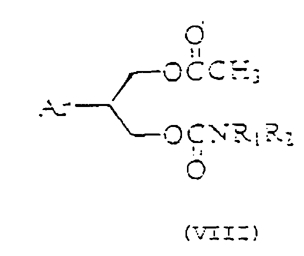 dans laquelle Ar, R₁ et R₂ ont la signification selon la revendication 1.

9. Carbamoyl (S)-3-N-substitué-2-aryl-propanol, représenté par la formule suivante IX : 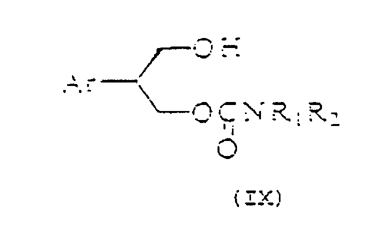 dans laquelle Ar, R₁ et R₂ ont la signification selon la revendication 1.

10. Sulfamate de (S)-3-acétoxy-2-aryl-1,3-propanediol, représenté par la formule suivante X : 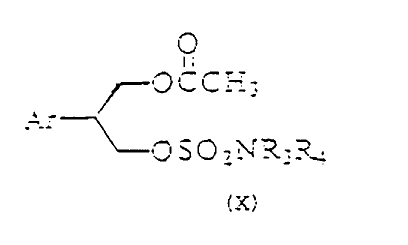 dans laquelle Ar, R₃ et R₄ ont la signification selon la revendication 1.

11. Monosulfamate de (S)-2-aryl-1,3-propanediol, représenté par la formule suivante XI : 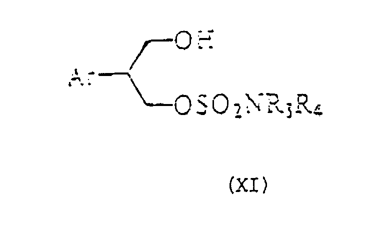 dans laquelle Ar, R₃ et R₄ ont la signification selon la revendication 1.

12. Acétate de carbamoyl R-3-N-substitué-2-aryl-propanol, représenté par la formule suivante XII : 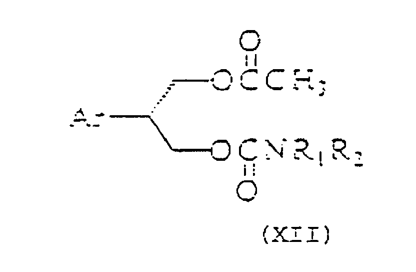 dans laquelle Ar, R₁ et R₂ ont la signification selon la revendication 1.

13. Carbamoyl R-3-N-substitué-2-aryl-propanol, représenté par la formule suivante XIII : 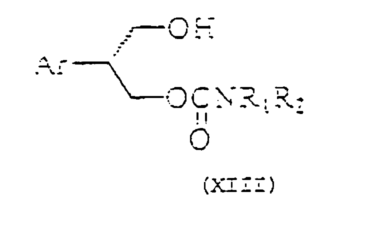 dans laquelle Ar, R₁ et R₂ ont la signification selon la revendication 1.

14. Un procédé de préparation de racémates de sulfamates de carbamoyl N,N'-substitué-2-aryl-propanol, représentés par la formule I ci-après, qui comprend les étapes de faire réagir du 3-acétoxy-2-aryl-propanol, représenté par la formule ci-après XIV, avec du carbonyl-diimidazole pour donner l'acétate de 3-imidazolyl-carbonyloxy-2-aryl-propanol, représenté par la formule XV ci-après, de faire réagir l'acétate de 3-imidazolyl-carbonyloxy-2-aryl-propanol avec une amine substituée, représentée par la formule suivante XVI, pour obtenir l'acétate de carbamoyl 3-N-substitué-2-aryl-propanol, représenté par la formule suivante IV, de soumettre l'acétate de carbamoyl 3-N-substitué-2-aryl-propanol à une transestérification dans un solvant alcoolique en présence d'un catalyseur basique pour obtenir un carbamoyl 3-N-substitué-2-aryl-propanol, représenté par la formule suivante V, et de faire réagir le carbamoyl 3-N-substitué-2-aryl-propanol avec du chlorure de sulfamoyle : 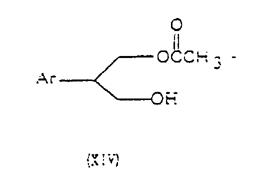 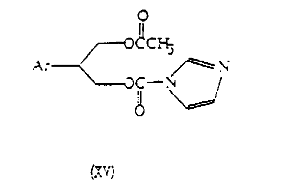
HNR₁R₂ (XVI) 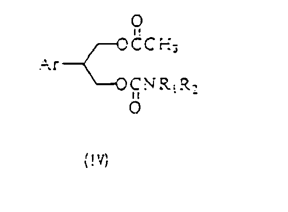 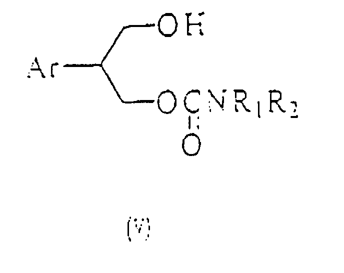 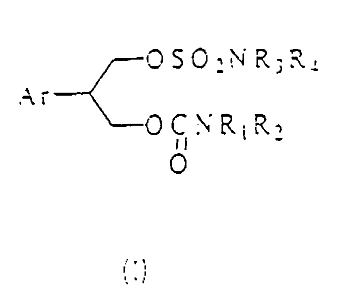
dans lesquelles
Ar est représenté par les formules suivantes : 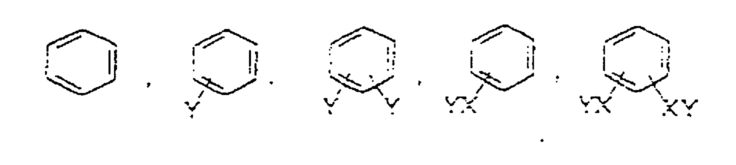
Y est choisi parmi le groupe comprenant les halogènes comme F, Cl, Br et I, les groupes trifluorométhyle et alkyle renfermant de 1 à 3 atomes de carbone lorsque Y seul est fixé au noyau de benzène et le groupe comprenant les radicaux trifluorométhyle et alkyle renfermant de 1 à 3 atomes de carbone lorsque Y est fixé à X qui est O ou S ; et
R₁, R₂, R₃ et R₄, qui sont identiques ou différents, sont chacun choisis parmi le groupe comprenant l'hydrogène, les radicaux alkyle linéaires ou ramifiés renfermant de 1 à 16 atomes de carbone, les groupes alkyle cycliques renfermant de 3 à 16 atomes de carbone et les groupes aryle renfermant de 6 à 8 atomes de carbone, et NR₁R₂ et NR₃R₄, qui sont identiques ou différents, peuvent former chacun un composé cyclique aliphatique de 3 à 7 membres, conjointement avec un autre atome d'azote ou atome d'oxygène.

15. Un procédé pour préparer un sulfamate de carbamoyl N,N'-substitué-2-aryl-propanol, représenté par la formule suivante I, qui comprend les étapes de faire réagir du 3-acétoxy-2-aryl-propanol, représenté par la formule suivante XIV, avec du chlorure de sulfamoyle en présence d'un catalyseur basique pour produire du sulfamate de 3-acétoxy-2-aryl-propanol, représenté par la formule suivante VI, de soumettre le sulfamate de 3-acétoxy-2-aryl-propanol à une transestérification dans un solvant alcoolique en présence d'un catalyseur basique pour produire un monosulfamate de 2-aryl-l,3-propanediol, représenté par la formule suivante VII, de faire réagir le monosulfamate de 2-aryl-1,3-propanediol avec du carbonyl-diimidazole pour obtenir un sulfamate de 3-imidazolyl-carbonyloxy-2-aryl-propanol, représenté par la formule suivante XVII, et de substituer le groupe imidazole du sulfamate de 3-imidazolyl-carbonyloxy-2-aryl-propanol avec une amine substituée, représentée par la formule suivante XVI : 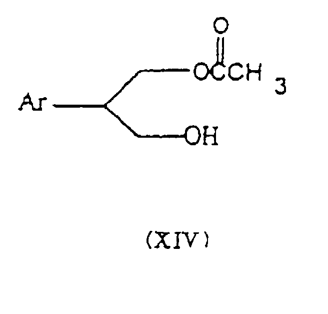 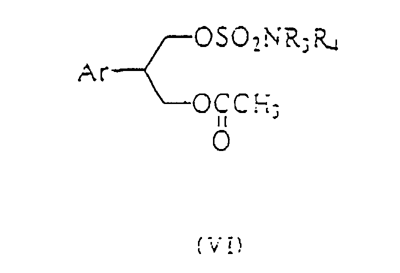 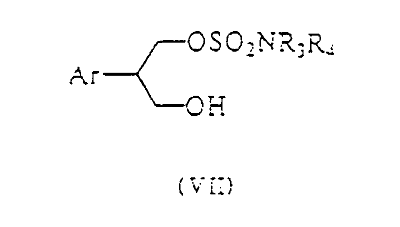 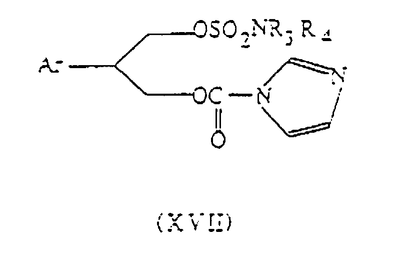
HNR₁R₂ (XVI) 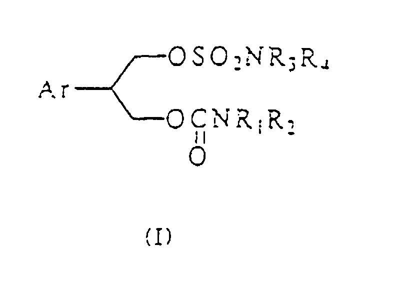
dans lesquelles Ar, R₁, R₂, R₃ et R₄ ont la signification selon la revendication 1.

16. Un procédé pour préparer un sulfamate de carbamoyl N,N'-substitué-2-aryl-1,3-propanediol, représenté par la formule suivante I, qui comprend les étapes de faire réagir un monosulfamate de 2-aryl-1,3-propanediol, représenté par la formule suivante VII, avec un isocyanate représenté par la formule suivante XVIII 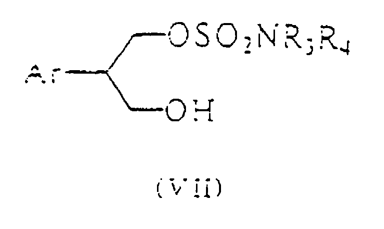
R₁NCO (XVIII) 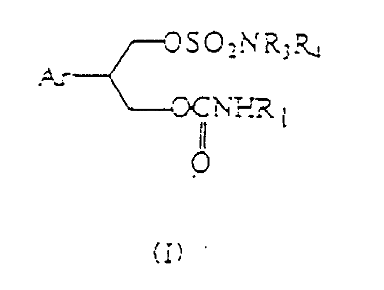
dans lesquelles Ar, R₁, R₃ et R₄ ont la signification selon la revendication 1.

17. Un procédé de préparation d'un sulfamate de carbamoyl R-N,N'-substitué-2-aryl-propanol, représenté par la formule suivante II, qui comprend les étapes de faire réagir du (R)-3-acétoxy-2-aryl-propanol, représenté par la formule suivante XIX, avec du carbonyl-diimidazole pour obtenir de l'acétate de (S)-3-imidazolyl-carbonyloxy-2-aryl-propanol, représenté par la formule suivante XX, de substituer le groupe imidazole de l'acétate de (S)-3-imidazolyl-carbonyloxy-2-aryl-propanol avec une amine substituée, représentée par la formule suivante XVI, pour obtenir un acétate de carbamoyl (S)-3-N-substitué-2-aryl-propanol, représenté par la formule suivante VIII, de soumettre l'acétate de carbamoyl (S)-3-N-substitué-2-aryl-propanol à une transestérification dans un solvant alcoolique en présence d'un catalyseur basique pour obtenir un carbamoyl (S)-3-N-substitué-2-aryl-propanol, représentée par la formule suivante IX, et de faire réagir le carbamoyl (S)-3-N-substitué-2-aryl-propanol avec du chlorure de sulfamoyle : 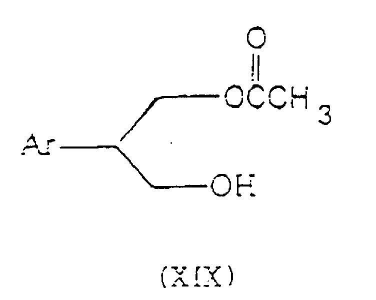 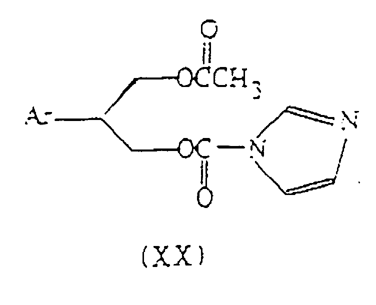
HNR₁R₂ (XVI) 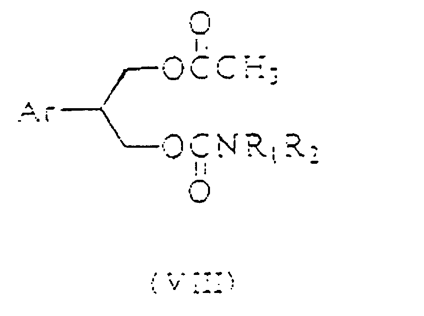 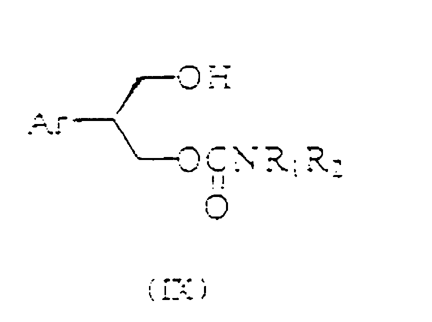 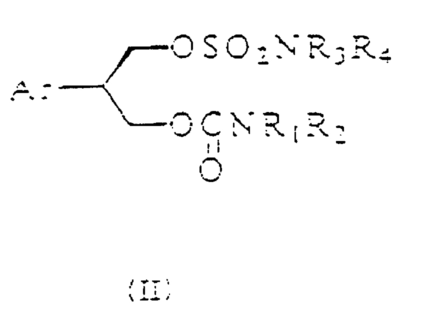
dans lesquelles Ar, R₁, R₂, R₃ et R₄ ont la signification selon la revendication 1.

18. Un procédé pour préparer un sulfamate de carbamoyl (S)-N,N'-substitué-2-aryl-propanol, représenté par la formule suivante III, qui comprend les étapes de faire réagir un (R)-3-acétoxy-2-aryl-propanol, représenté par la formule suivante XIX, avec du chlorure de sulfamoyle en présence d'un catalyseur basique pour produire un sulfamate de (S)-3-acétoxy-2-aryl-1,3-propanediol, représenté par la formule suivante X, de soumettre le sulfamate de (S)-3-acétoxy-2-aryl-l,3-propanediol à une transestérification dans un solvant alcoolique en présence d'un catalyseur basique pour obtenir un monosulfamate de (S)-2-aryl-1,3-propanediol, représenté par la formule suivante XI, de faire réagir le monosulfamate de (S)-2-aryl-1,3-propanediol avec du carbonyl-diimidazole pour obtenir un sulfamate de (S)-3-imidazolyl-carbonyloxy-2-aryl-propanol, représenté par la formule suivante XXI, et de substituer le groupe imidazole du sulfamate de (S)-3-imidazolyl-carbonyloxy-2-aryl-propanol avec une amine substituée, représentée par la formule suivante XVI : 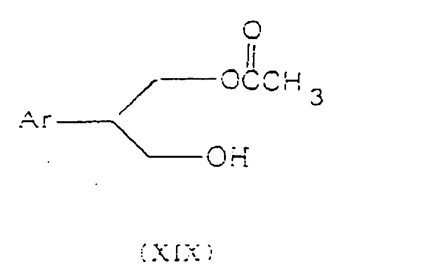 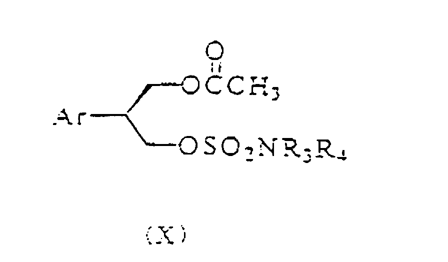 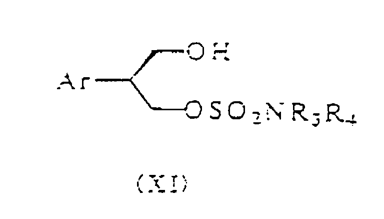 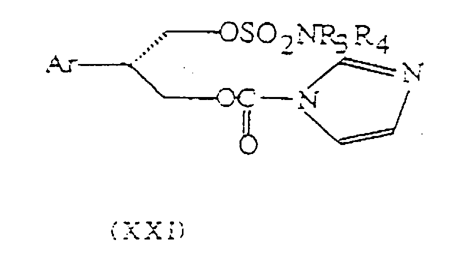
HNR₁R₂ (XVI) 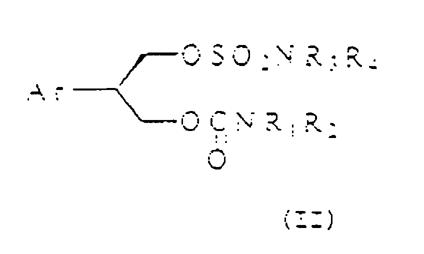
dans lesquelles Ar, R₁, R₂, R₃ et R₄ ont la signification selon la revendication 1.

19. Un procédé pour préparer un sulfamate de carbamoyl (S)-N,N'-substitué-2-aryl-propanol, représenté par la formule suivante III, qui comprend les étapes de faire réagir un (S)-3-acétoxy-2-aryl-propanol, représenté par la formule suivante XXII, avec du carbonyl-diimidazole pour obtenir un acétate de (R)-3-imidazolyl-carbonyloxy-2-aryl-propanol, représenté par la formule suivante XXIII, de substituer le groupe imidazole de l'acétate de (R)-3-imidazolyl-carbonyloxy-2-aryl-propanol avec une amine substituée, représentée par la formule suivante XVI, pour obtenir un acétate de carbamoyl (R)-3-N-substitué-2-aryl-propanol, représenté par la formule suivante XII, de soumettre l'acétate de carbamoyl (R)-3-N-substitué-2-aryl-propanol à une transestérification dans un solvant alcoolique en présence d'un catalyseur basique pour obtenir du carbamoyl (R)-3-N-substitué-2-aryl-propanol, représenté par la formule suivante XIII, et de faire réagir le carbamoyl (R)-3-N-substitué-2-aryl-propanol avec du chlorure de sulfamoyle : 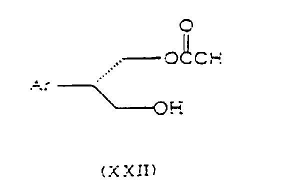 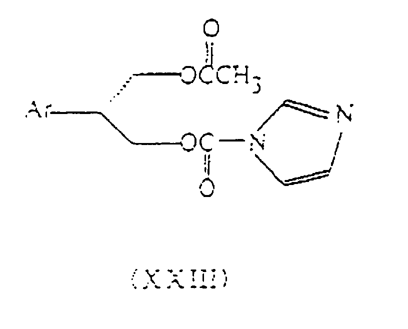
HNR₁R₂ (XVI) 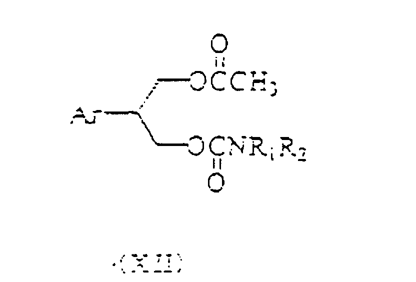 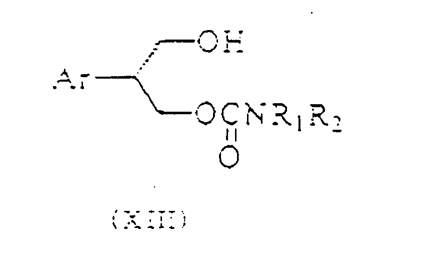 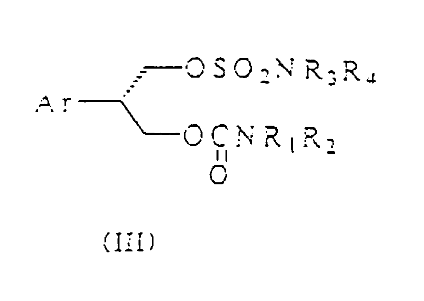
dans lesquelles Ar, R₁, R₂, R₃ et R₄ ont la signification selon la revendication 1.

20. Une composition pharmaceutique pour traiter les désordres du système nerveux central qui comprend, comme ingrédient actif, une quantité efficace pour traiter les désordres du système nerveux central d'un composé de formule (I) comme défini dans la revendication 1, de formule (II) comme défini dans la revendication 2, ou de formule (III) comme défini dans la,revendication 3, et un véhicule (« carrier ») acceptable du point de vue pharmaceutique.

21. La composition selon la revendication 20, dans laquelle le désordre du système nerveux central qui est traité est choisi parmi le groupe comprenant les convulsions et l'épilepsie.
